(19) Europäisches Patentamt
European Patent Office
Office européen des brevets

(11) Numéro de publication: **0 008 556**
**B1**

# (12) FASCICULE DE BREVET EUROPEEN

(45) Date de publication du fascicule du brevet: **25.01.84**

(21) Numéro de dépôt: **79400561.1**

(22) Date de dépôt: **09.08.79**

(51) Int. Cl.³: **C 07 D 471/04,** A 61 K 31/435 // (C07D471/04, 221/00, 209/00),(C07D401/06), (C07D209/42),(C07D209/14), (C07D209/18)

(54) **Dérivés de bis 7H pyridocarbazoles et composition pharmaceutique les contenant.**

(30) Priorité: **11.08.78 FR 7823801**

(43) Date de publication de la demande: **05.03.80 Bulletin 80/5**

(45) Mention de la délivrance du brevet: **25.01.84 Bulletin 84/4**

(84) Etats contractants désignés: **BE CH DE FR GB IT NL SE**

(56) Documents cités:
**COMPTES RENDUS DES SEANCES DE L'ACADEMIE DES SCIENCES, T.284 (1977), série D; pages 81-84**
**CHEMICAL ABSTRACTS, vol. 82, (1975), abrégé 106193y, page 15. Columbus, Ohio, USA**
***CHEMICAL ABSTRACTS, vol. 85, (1976) abrégé 88896k,* page 191. Columbus, Ohio, USA**

**Le dossier contient des informations techniques présentées postérieurement au dépôt de la demande et ne figurant pas dans le présent fascicule.**

(73) Titulaire: **ANVAR Agence Nationale de Valorisation de la Recherche, 13, rue Madeleine Michelis, F-92522 Neuilly-sur-Seine (FR)**

(72) Inventeur: **Roques, Bernard P., 12, rue Eugène Delacroix, F-94410 Saint Maurice (FR)**
Inventeur: **Le Pecq, Jean-Bernard, 37, Square Saint-Charles, F-75012 Paris (FR)**
Inventeur: **Pelaprat, Didier, 136, rue de Paris, F-94220 Charenton (FR)**
Inventeur: **Le Guen, Irène, néeRolle, 205, Boulevard Raspail, F-75014 Paris (FR)**

(74) Mandataire: **Gutmann, Ernest et al, Cabinet Plasseraud 84, rue d'Amsterdam, F-75009 Paris (FR)**

**EP 0 008 556 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Dérivés de bis 7 H pyridocarbazoles et composition pharmaceutique les contenant

L'invention est relative à des composés nouveaux ayant des propriétés antitumorales et qui mettent en œuvre la propriété que possèdent certaines molécules de s'intercaler dans des acides désoxyribonucléiques ou ADN.

La structure de l'ADN est bien connue. Elle est constituée de deux chaînes de nucléotides, en hélice, chacune de ces chaînes comprenant une armature formée par des molécules de désoxyribose reliées entre elles par des ponts phosphodiesters. A ces chaînes sont reliées des bases qui s'apparient par paires (adénine-thymine et guanine-cytosine) de chaîne à chaîne, par l'intermédiaire de liaisons hydrogène. Ces appariements contribuent à la formation de la structure en double hélice caractéristique des ADN.

Il a déjà été constaté que certaines molécules étaient capables de s'intercaler dans l'ADN notamment dans des espaces ou «loges» susceptibles de se former entre des paires de bases voisines de cet ADN, dans la mesure où ces molécules étrangères possèdent à la fois une configuration moléculaire et une affinité suffisantes pour ces paires de bases.

Diverses molécules de ce type ont déjà été décrites. En particulier, il s'est avéré que des composés tels que le bromure d'éthidium, des sels de phénanthridinium et d'acridine présentaient une planéité suffisante pour pénétrer dans ces espaces ou loges, lesquelles présentent une dimension de l'ordre de 3,4 Å dans la direction de l'axe théorique d'enroulement de la double hélice (distance entre deux paires voisines de bases dans la structure en hélice de l'ADN).

De même, a été constatée la capacité de certains antibiotiques, notamment de l'actinomycine et de la daunomycine de s'intercaler ainsi dans des chaînes d'ADN.

Le mécanisme de l'intercalation de ces composés entre des paires de bases de l'ADN a été décrit dans de nombreuses publications. A titre d'exemple, on peut relever que le modèle de l'intercalation a été confirmé par les études cristallographiques de Jain et Sobell sur l'actinomycine D (J. Mol. Biol. 68, 1972, p 1 et 21) et sur le bromure d'éthidium (Proc. Nat. Acad. Sc., USA, 72, 1975, p. 628).

Par ailleurs, il est bien connu que les ellipticines et leurs dérivés présentent une activité thérapeutique contre la leucémie murine L 1210. C'est ce qui figure dans l'article des C. R. Acad. Sc. Paris, t. 284 (1977), série D, p. 81–84, dans lequel est mentionné que le procédé de dimérisation à l'instar de ce qui a été obtenu avec les acridines et l'éthydium permet d'atteindre des affinités de l'ordre de celles des substrats biologiques pour l'ADN.

Mais l'hypothèse formulée par les auteurs de cet article et selon laquelle l'établissement d'une liaison entre deux noyaux aromatiques par des chaînes amidoalcoylées pourrait accroître l'activité thérapeutique de ces composés, s'est révélé inexacte.

En effet, la dimérisation des monomères de 6 H pyridocarbazoles par l'intermédiaire de chaînes a entraîné une réduction des propriétés antitumorales vis-à-vis de celles que possédaient initialement des monomères de 6 H pyridocarbazoles rendant par là toute anticipation impossible vis-à-vis de la relation entre les structures générales de classes nouvelles de produits et leurs propriétés biologiques.

Or, la Société Demanderesse a trouvé de façon surprenante qu'en dimérisant, par l'intermédiaire de chaînes appropriées, des molécules de 7 H pyridocarbazoles, lesquelles ne présentent elles-mêmes qu'une activité antitumorale très réduite, sinon nulle, contrairement à ce que l'on observe pour les 6 H pyridocarbazoles, on obtient des dimères actifs vis-à-vis de la leucémie L1210.

La classe de composés selon la présente invention est constituée par les sels d'ammonium quaternaire de dérivés de bis 7 H pyridocarbazoles de formule suivante:

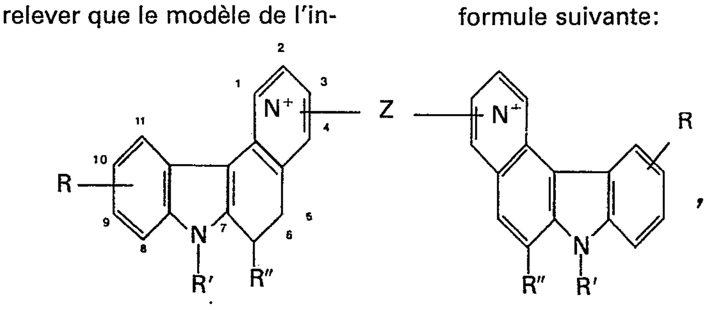
, $2A^-$

(1)

dans laquelle:

l'atome d'azote du cycle pyridile occupe l'une quelconque des positions 1 à 4; et

Z est une chaîne moléculaire semi-rigide ou rigide, comprenant des groupements aminoalkyle ou aminocyclique, le cas échéant aussi des groupes alkyle, hydroxyalkyle ou hydroxyaryle de formule:

$$-(CH_2)_n - W - (CH_2)_{n'}-$$

dans laquelle:

n et n' varient de 2 à 4;

W est choisi parmi les groupes suivants:

$-(CH_2)_m-$

$-NH-(CH_2)_p-(CHOH)_m-(CH_2)_p, -NH-$

$-NR_1-(CH_2)_p-(CHOH)_m-(CH_2)_p, -NR_1-$

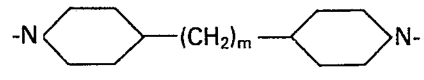

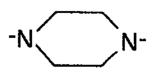

avec m variant de 0 à 4, et p, p' prenant les valeurs 1 ou 2;

$-NH-(CH_2)_{m'}-NH-$

$-NR_1-(CH_2)_{m'}-NR_1-$

avec m' variant de 2 à 8;

le 1,4-diamine benzène,

le groupe 4,4'-diaminobiphényle,

la 4,4'-bipyridine,

le groupe décahydronaphthyridine,

$R_1$ est un groupe alkyle comprenant de 1 à 10 atomes de carbone, de préférence méthyle ou éthyle.

R qui occupe une position quelconque sur le noyau correspondant est un hydrogène, un halogène, un groupe nitro, un groupe amino, le cas échéant substitué par des restes alkyle comprenant de 1 à 10 atomes de carbone, ou des restes acyle comprenant de 2 à 10 atomes de carbone ou de 7 à 10 atomes de carbone lorsque ce sont des restes aroyle, par des restes aryle comprenant de 6 à 10 atomes de carbone, un groupe OH, le cas échéant substitué par des restes alkyle comprenant de 1 à 12 atomes de carbone, acyle comprenant de 2 à 12 atomes de carbone ou de 7 à 12 atomes de carbone lorsque ce sont des restes aroyle, par des restes aryle comprenant de 6 à 12 atomes de carbone;

R' est un hydrogène, un reste alkyle comprenant de 1 à 10 atomes de carbone ou un reste aryle comprenant de 6 à 12 atomes de carbone, ou acyle comprenant de 2 à 10 atomes de carbone ou de 7 à 12 atomes de carbone lorsqu'il s'agit d'un groupe aroyle;

R" est un atome d'hydrogène,

un radical cyano, formyle, hydroxyméthyle éventuellement estérifié par un acide-carboxylique aliphatique de 1 à 10 atomes de carbone;

un radical aminométhyle éventuellement substitué sur l'amino par un groupe alkyle ou acyle de 1 à 10 atomes de carbone;

un radical carboxyle éventuellement estérifié par un alcool aliphatique de 1 à 10 atomes de carbone;

ou un radical amido;

$A^-$ étant l'anion d'un acide physiologiquement acceptable.

En ce qui concerne l'anion $A^-$ associé au dérivé de bis 7 H pyridocarbazole, il est dérivé de préférence d'un acide physiologiquement acceptable et est notamment un halogénure tel que $Cl^-$, $Br^-$, $I^-$, un acétate, un anion organique ou minéral capable d'exalter la solubilité dans l'eau ou la pénétration membranaire, en particulier le lactate, le gluconate, l'isothionate.

Les composés conformes à l'invention ont une action antitumorale tout à fait remarquable, supérieure pour certains d'entre eux aux médicaments antitumoraux utilisés à l'heure actuelle, dans un certain nombre d'essais pharmacologiques dont la validité et la corrélation à l'activité en thérapeutique sont à ce jour bien reconnus des spécialistes.

Les dérivés de bis 7 H pyridocarbazoles selon l'invention autorisent la bis-intercalation dans une molécule d'ADN. La chaîne reliant les deux groupes 7 H pyridocarbazoles possède une structure moléculaire telle que les plateaux 7 H pyridocarbazole sont maintenus sensiblement parallèles l'un par rapport à l'autre, et ce en l'absence de possibilités d'auto-association. Elle possède aussi de préférence une longueur telle que la distance entre les plans médians des plateaux soit approximativement égale à un multiple n de 3,4 Å, n étant un nombre notamment de 1 à 6, ou de préférence de 2, 3 ou 4.

Le phénomène de bis-intercalation pourra être mieux compris grâce aux figures 1a, 1b et 1e sur lesquelles est représentée une molécule d'ADN. Les paires de bases et la double hélice schématisées sur les figures 1a, 1b et 1e sont respectivement désignées par PB et H. Les dérivés de bis 7 H pyridocarbazoles sont schématisés par des plateaux de groupes 7 H pyridocarbazoles désignés par M, réunis par une chaîne rigide ou semi-rigide, désignée par Z et représentée par une ligne brisée sur les figures 1c et 1d.

La molécule d'ADN pouvant subir des déformations par un mécanisme de respiration laisse apparaître par distorsion des loges désignées par L comme le montre notamment la figure 1b, disposées de façon aléatoire entre les paires de bases PB, loges dans lesquelles les dérivés de bis 7 H pyridocarbazoles selon la présente invention peuvent se bis-intercaler, notamment comme dans le cas de la figure 1e, chacun des plateaux M des dérivés de bis 7 H pyridocarbazoles s'insérant dans une loge L.

Les figures 1e et 1b représentent un cas particulier pour lequel deux loges consécutives, prises au hasard sur la molécule d'ADN sont séparées par deux paires de bases adjacentes, ceci n'excluant evidemment pas le cas où deux loges consécutives sont séparées par une paire de bases ou par plus de deux paires de bases, ce qui explique les conditions ci-dessus énoncées auxquelles doit répondre la chaîne pour que la bis-intercalation soit possible.

Les dérivés de bis 7 H pyridocarbazoles de la présente invention sont tels que l'auto-association est évitée. Cette auto-association représentée sur la figure 1d résulte des forces d'attraction mutuelles exercées par chacun des plateaux, l'un sur l'autre, l'attraction entre les deux plateaux d'un dérivé de bis 7 pyridocarbazoles étant suffisante pour aboutir à un empilement des 2 plateaux.

Selon la présente invention, les dérivés de bis 7 H pyridocarbazoles sont également tels que les deux plateaux se trouvent maintenus dans un état de relatif parallélisme, comme il est représenté sur la figure 1c, et non pas dans une position incompatible avec les conditions de bis-intercalation.

Une des classes de composés préférés, selon la présente invention, est représentée par les sels d'ammonium quaternaire de dérivés de bis 7 H pyridocarbazoles de formule suivante:

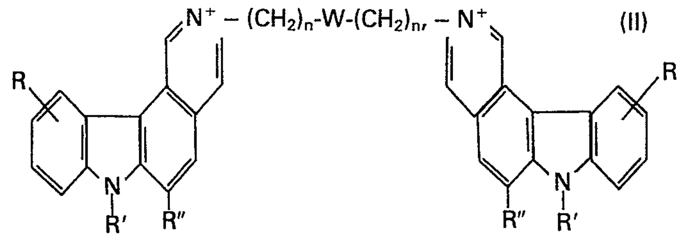

(II) , $2A^-$

dans laquelle n et n' sont égaux à 2 ou 3, et R représente un méthoxy ou un hydroxy.

Une autre classe de composés préférés selon l'invention, est constituée par les sels d'ammonium quaternaire de dérivés de bis 7 H pyridocarbazoles de formule (I) ci-dessus indiquée, dans laquelle:

l'atome d'azote est en position 2,

R est en position 10 et représente l'un des groupes suivants: H, OH, Br, $OCH_3$, $OCOCH_3$, $OCOCH_2CH_3$, $O(CH_2)_2CH_3$, $NO_2$, $NH_2$;

R' représente H ou $CH_3$;

R" représente l'un des groupes suivants: H, CN, $CONH_2$, COOH, $CH_2NH_2$.

Une autre classe de composés préférés selon l'invention, est constituée par les sels d'ammonium quaternaire de dérivés de bis 7 H pyridocarbazoles de formule (III) suivante:

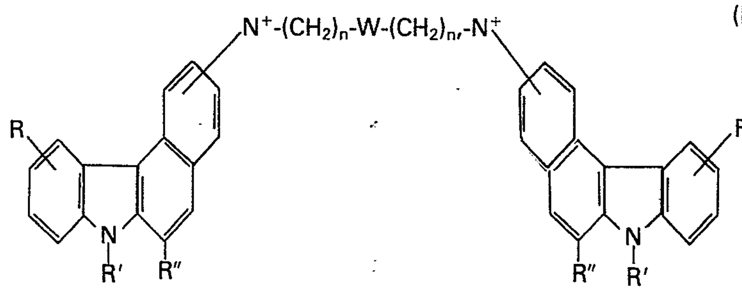

(III) , $2A^-$

dans laquelle notamment:

R, de préférence en position 10, représente le groupe méthoxy ou hydroxy,

n et n', de préférence égaux, prennent la valeur 2 ou 3,

W est représenté par la formule X-Y-X dans laquelle:

X représente la pipéridine et Y est le groupe $(CH_2)_3$ ou, X représente le groupe NH et Y est le groupe $(CH_2)_6$ ou le groupe $CH_2$-$(CHOH)_4$-$CH_2$.

Une autre classe de composés préférés selon l'invention est constituée par les sels d'ammonium quaternaire de dérivés de bis 7 H pyridocarbazole de formule (IV) suivante:

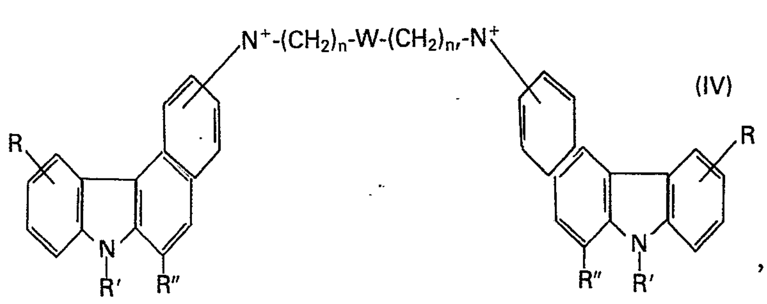

(IV) , $2A^-$

dans laquelle:

R, de préférence en position 10, représente un reste méthoxy ou hydroxy,

R' représente un atome d'hydrogène ou un reste méthyle,

R" représente un atome d'hydrogène,

W représente:

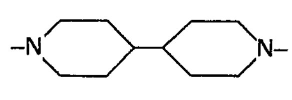

n et n', de préférence égaux, prennent la valeur 2 ou 3.

Des composés particulièrement préférés selon l'invention répondent à l'une des formules suivantes:

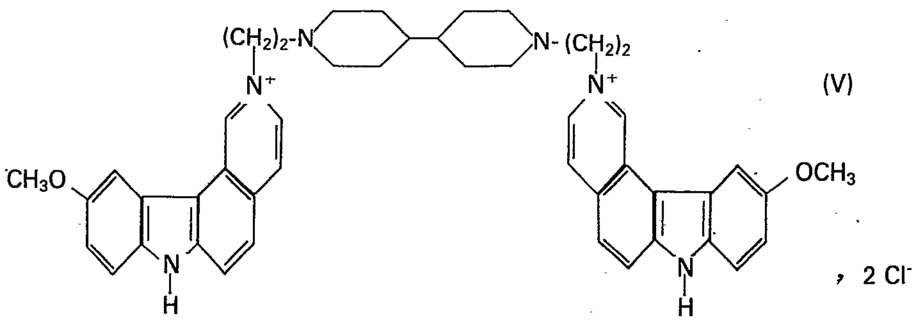

(V)

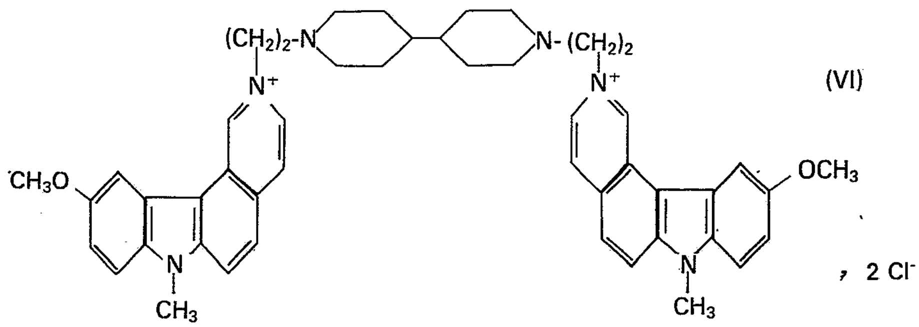

(VI)

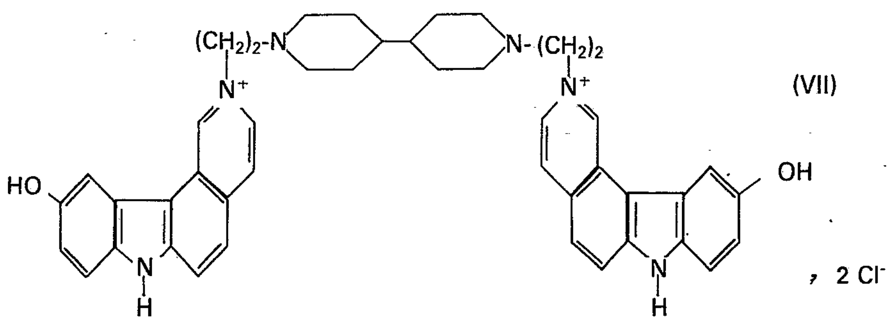

(VII)

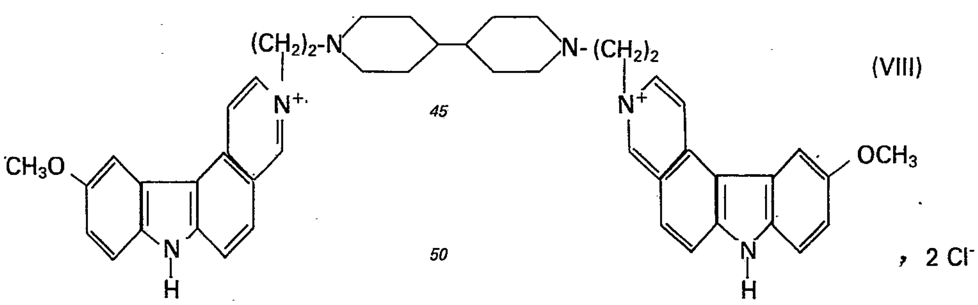

(VIII)

La synthèse des dimères de pyridocarbazoles envisagés dans la présente invention va être exposée dans ce qui suit. Cette synthèse a lieu en deux étapes, la première étant l'obtention du groupe 7 H pyridocarbazole, la seconde étant la dimérisation.

La première étape est relative à l'obtention de groupes de formule:

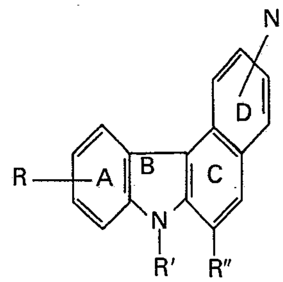

(IX)

dans laquelle les cycles A et B correspondant à un dérivé indolique, sont condensés avec un composé comportant le cycle D (dérivé pyridinique).

Le cycle C est ensuite fermé par photocyclisation. Les étapes successives aboutissant au tétracycle de base sont indiquées sur un exemple décrit ci-dessous dans lequel R représente le groupement méthoxy, R' et R" étant des atomes d'hydrogène.

Le produit de départ est l'acide indole-2 carboxylique substitué en 7 par le groupement méthoxy de formule:

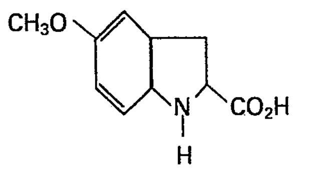
(X)

sur lequel on fait réagir un chlorure d'acide minéral, ce qui conduit à l'obtention du chlorure de l'acide méthoxy-5 indole-2 carboxylique, qui est ensuite transformé en N,N-diméthylamide; cet amide est ensuite réduit en amine par LiA1H4, l'amine est quaternarisée par l'iodure de méthyle, et l'ammonium quaternaire est substitué par la triphénylphosphine, ce qui conduit au sel de phosphonium de l'indole. Celui-ci est ensuite mis en réaction selon la méthode de Wittig avec l'aldéhyde pyridinique adéquat, pour donner les cis et trans indolylpyridyléthylènes correspondants, respectivement de formule (XI) et (XII):

cis

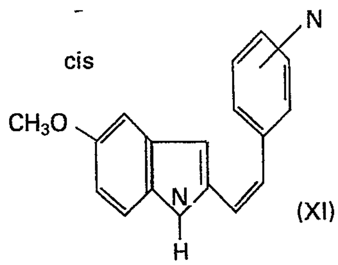
(XI)

trans

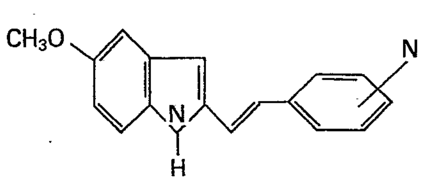
(XII)

Ceux-ci sont enfin transformés en pyridocarbazoles par photocyclisation oxydative selon les méthodes de O. De Silva et coll. Synthésis (1971) 254–255 et D. Pellaprat et coll. C. R. Acad. Sci., série D, 283 (1976) 1109.

L'introduction des divers substituants sur les tétracycles s'effectue d'une part en employant les indoles convenablement substitués, d'autre part en introduisant ces substituants a posteriori sur le cycle lui-même.

L'acide indole-2 carboxylique est un produit commercial de formule:

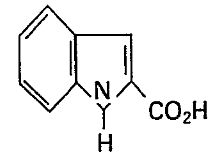
(XIII)

Tous les autres dérivés indoliques sont préparés selon des techniques décrites dans la littérature; par exemple, dans le cas de l'acide méthoxy-7-indole de formule:

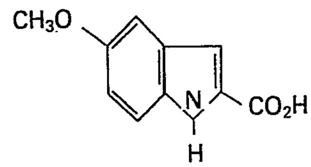
(X)

le produit de départ est la paraméthoxy aniline, que l'on soumet à une nitrosation. Le sel de diazonium obtenu, par action du méthyl-2-malonate d'éthyle, en présence d'acétate de sodium conduit au dérivé de formule:

(XIV)

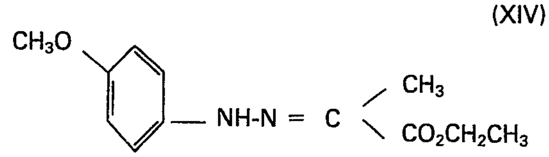

que l'on cyclise en milieu acide, en présence d'éthanol, ce qui donne après saponification par la potasse, l'acide méthoxy-7 indole.

Introduction des radicaux R" en position 6:

Les radicaux R" en position 6 dérivent tous du radical CN introduit au cours de la synthèse par action du cyanure de potassium sur un iodure de triméthyle ammoniométhyl-2 indole substitué ou non sur l'indole par un radical R, notamment en position 5. Le composé obtenu est condensé avec une formylpyridine pour conduire au mélange des éthyléniques cis et trans de formule:

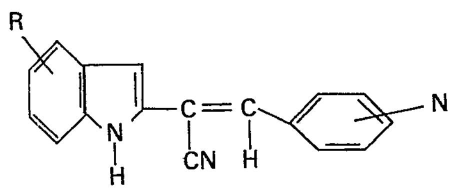
(XV)

N en position 1, 2, 3 ou 4 composés cis et trans

Ceux-ci sont photocyclisés comme précédemment pour donner le composé de formule:

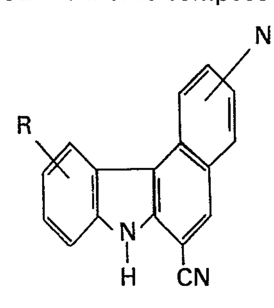
(XVI)

N en position 1, 2, 3 ou 4

Le reste CN est alors transformé notamment en aldéhyde, alcool, acide, amide ou aminométhyl par les méthodes classiques.

Introduction des radicaux R en position 10:

L'introduction du radical R en position 10 sur le tetracycle peut se faire par voie directe ou indirecte.

Par voie directe, on peut introduire le groupement nitro, $NO_2$, le groupement amino, $NH_2$, seul ou substitué par un radical acyle ou aroyle, alkyle ou aryle.

Par voie indirecte, on peut introduire le groupement hydroxyle, seul ou substitué par un radical acyle ou alcoyle, alkyle ou aryle.

Les composés obtenus par voie directe s'obtiennent tous en partant du tétracycle de formule:

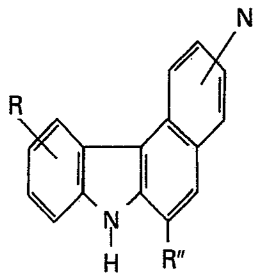

(XVII)

N en position 1, 2, 3 ou 4

Une nitration de ce composé introduit le groupement nitro, $NO_2$. Puis par réduction du groupement nitro, on peut obtenir le groupement amino, $NH_2$.

Une réaction d'acylation sur le groupement $NH_2$ permet de le substituer par un radical acyle ou aroyle. Le substituant R devient alors soit:

$(CH_3)-(CH_2)_n-\underset{\underset{O}{\|}}{C}-NH$, soit $Ar-(CH_2)_2-\underset{\underset{O}{\|}}{C}-NH$,

Ar représentant un composé aromatique.

En halogénant le tétracycle non substitué, on peut introduire un halogène, par exemple le brome en position 10. Le composé halogéné, soumis à une amination, est alors substitué en 10 par un radical de type $CH_3-(CH_2)_n-NH$ ou $Ar-(CH_2)_n-NH$, selon la nature du corps qui intervient dans l'amination.

Les composés obtenus par voie indirecte s'obtiennent tous à partir du composé de formule:

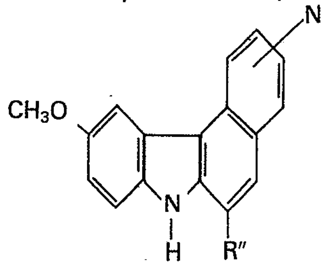

(XVIII)

N en position 1, 2, 3 ou 4

L'action d'acide bromhydrique ou du mélange acide chlorhydrique-pyridine conduit au composé hydroxylé de formule:

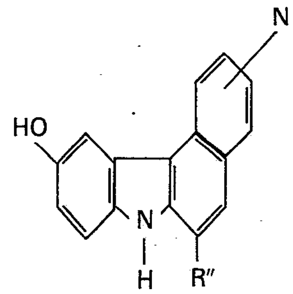

(XIX)

N en position 1, 2, 3 ou 4

Ce composé peut être acylé, et selon le groupe acylant, le substituant en 10 sera:

$CH_3-(CH_2)_n-\underset{\underset{O}{\|}}{C}-O$ ou $AR-(CH_2)_n\underset{\underset{O}{\|}}{C}-O$

Ce composé peut également être éthérifié. Le substituant en 10, selon les corps utilisés dans l'éthérification, aura la formule suivante:

$CH_3-(CH_2)_n-O$ ou bien $AR-(CH_2)_n-O$

Il faut noter que les introductions possibles des groupements R que l'on vient de décrire se font par des moyens de synthèse classiques.

Pour conduire aux dérivés de bis 7 H pyridocarbazoles selon l'invention, la dimérisation se fait par réaction de deux équivalents de l'un des groupes 7 H pyridocarbazoles décrits précédemment, dans un solvant aprotique permettant de se trouver dans des conditions opératoires à des températures élevées, et qui réduit les risques de polymérisation et dans lequel le dimère ne se solubilise pas, ce qui permet au dérivé de bis 7 H pyridocarbazole de précipiter. Parmi ces solvants, on peut citer le diméthylacétamide, le diméthylsulfoxyde, la N-pyrrolidone, le diméthylformamide. La réaction a lieu à une température d'environ 80°C à environ 120°C, de préférence d'environ 95°C à environ 105°C et avantageusement à environ 100°C, durant environ 10 à environ 20 heures, de préférence environ 16 heures, sur un équivalent d'une des chaînes de formule:

$$T-(CH_2)_n-X-Y-X'-(CH_2)_{n'}-T$$

dans laquelle T est choisi dans le groupe comprenant le chlore, le brome, l'iode ou le groupe tosyle.

Cette chaîne est obtenue notamment:

commercialement dans le cas de la 4,4'-(chloroéthyl)-bis-pipéridine et dans le cas du 1,6-bis(2-chloroéthylamino)-1,6-dideoxy-D-mannotol (degranol),

par synthèse selon les méthodes décrites dans le brevet UK 1173244 (1969), ainsi que les méthodes décrites par Bruneau et al., Bull. Soc. Chim. France (1968) n° 8, p. 3275,

en condensant successivement les composés de type $HO'(CH_2)_n$-T et $HO(CH_2)_n$-T sur le composé de type X-Y-X'; où le groupe X-Y-X' est avantageusement constitué par une diamine aliphatique (d'éthylène diamine à octane diamine), une diamine alicyclique (pipérazine, 4,4'-bis-pipéridine ou bis-pipéridyl-alkanes, une amine aromatique (1,4-diamine benzène, 4,4'-diaminobiphényl) ou hétérocycle (4,4'-bipyridine, bipyridylalcanes, décahydronaphtyridines), ce qui conduit au dérivé $HO-(CH_2)_n-X-Y-X'-(CH_2)_{n'}-OH$,

en condensant le groupe X-Y-X' tel que défini précédemment sur l'oxyde d'éthylène ou l'oxyde de triméthylène selon la technique décrite dans J. Med. Pharm. Chem. (1959), 1, p 223 et dans J. Med. Chem. D. Pelaprat et al, in préparation.

Ce composé est ensuite par exemple chloré par action du chlorure de thionyle, pour donner la chaîne:

$$Cl-(CH_2)_n-X-Y-X'-(CH_2)_{n'}-Cl.$$

Outre ceux précédemment décrits, les produits nécessaires aux synthèses sont connus ou peuvent être préparés et purifiés selon les méthodes connues à partir de composés connus ou selon les procédés décrits dans les exemples.

Les exemples 1 à 11 sont relatifs à la synthèse des groupes 7 H pyridocarbazoles et les exemples 12 à 25 sont relatifs à la synthèse des sels d'ammonium quaternaire des dérivés de bis 7 H pyridocarbazoles obtenus à partir des 7 H pyridocarbazoles.

Synthèse des 7 H pyridocarbazoles

Exemple 1

Synthèse du méthoxy-10, 7-H-pyrido[4,3-c]carbazole, dérivé 1.

$R = OCH_3$, R' = H, R" = H

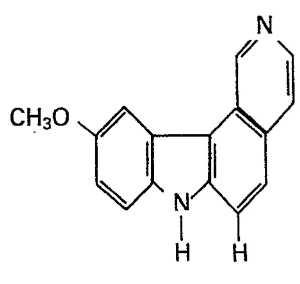

Etape a. N,N-diméthyl méthoxy-5 indolamide-2

A une suspension de 17,1 g (89,5 mM) d'acide 5-méthoxy indole-2 carboxylique dans 245 ml de benzène, on introduit en 10 mn et à 20°C 23,8 ml (335 mM) de $SOCl_2$ purifié. On laisse 10 minutes sous agitation puis chauffe 2 h 30 à 57°C. La solution devient limpide, puis se trouble et précipite.

On laisse reposer 3 heures. On essore, rince avec 50 ml de benzène. Puis on dissout le solide jaune obtenu dans 260 ml d'éther anhydre. Les eaux-mères benzéniques sont concentrées, et le solide jaune obtenu est également dissout dans l'éther. Un résidu noirâtre reste sur le fritté. Les solutions éthérées sont groupées et ajoutées en 45 mn, à 25–27°C, à 60 ml de diméthylamine en solution benzénique à 33%. On laisse 15 mn sous agitation et ajoute 160 ml d'eau. On essore, rince à l'eau avec empatage jusqu'à pH neutre des eaux de lavage. On sèche sous vide.

Pds: 15,4 g; Rdt: 79%; F: 207°C.

Etape b. N,N-diméthyl aminométhyl-2 méthoxy-5 indole

On ajoute en 5 mn à 10–15°C, 113 ml de tétrahydrofuranne (THF) anhydre, 5,65 g (145 mM) d'hydrure d'aluminium lithium, on retire ensuite le bain réfrigérant et additionne en 45 mn, 14,3 g de N,N-diméthylméthoxy-5 indolamide-2 (65,5 mM) en suspension dans 385 ml de THF. La température monte à 35°C; on maintient 3 heures à 45–50°C, puis on refroidit à −10°C et on ajoute 85 ml d'eau. On essore, lave au THF, évapore les eaux-mères et de lavage, ajoute 23 ml de soude 2N au résidu mi-aqueux mi-huileux. On extrait 2 fois à l'éther, lave 2 fois à l'eau, une fois avec une solution saturée de chlorure de sodium, sèche sur sulfate de sodium, filtre et évapore sous vide.

Pds: 13,35 g; Miel brun; Rdt: 100% (brut).

Etape c. Iodure de triméthylammoniométhyl-2-méthoxy-5 indole

A une solution de 12,8 g de N,N-diméthylaminométhyl-2 méthoxy-5 indole (62,8 mM) dans 55 ml d'acétate d'éthyle, on ajoute en 30 mn goutte à goutte une solution de 4,3 ml d'iodure de méthyle (1,1 × 62,8 mM) dans 27,5 ml d'acétate d'éthyle. On laisse 1 heure à 45–55°C sous agitation, puis laisse reposer 2 heures à température ambiante. On essore, rince avec 2 fois 10 ml d'acétate d'éthyle, sèche sous vide.

Pds: 19,25 g; F: 184°C. On recueille un deuxième jet en concentrant les eaux-mères.

Pds: 850 mg; F: 184°C; Rdt: 92%.

Etape d. Iodure de triphénylphosphoniométhyl-2 méthoxy-5 indole

Dans 190 ml de diméthylformamide (DMF) anhydre, on verse 20,1 g (58 mM) d'iodure de triméthylammoniométhyl-2 méthoxy-5 indole et 20 g (1,31 × 58 mM) de triphénylphosphine. On porte cette solution en une heure à 120–130°C, laisse 24 heures à 130°C sous agitation et azote, puis une nuit à température ambiante.

On évapore ensuite le DMF sous vide, ajoute du benzène à l'huile obtenue et laisse proliférer les cristaux. On essore, rince avec 2 fois 10 ml de benzène, sèche sous vide.

Pds: 24,6 g; F: 259–160°C; Rdt: 77%.

Etape e. 1-[2-(méthoxy-5) indole]-2-(4-pyridyl) éthylène cis et trans

On dissout à 60°C 8,1 g (14,75 mM) d'iodure de triphénylphosphoniométhyl-2 méthoxy-5 indole dans 250 ml de méthanol, puis on ajoute simultanément à 65°C, 64 ml de méthylate de sodium (360 mg de sodium dans 64 ml de méthanol) et 1,7 ml (18 mM) de formyl-4 pyridine dans 30 ml de méthanol. On laisse 24 heures sous azote et agitation. On évapore le méthanol jusqu'à 400 ml et laisse proliférer les cristaux.

Pds: 1,435 g; F: 252°C. On obtient un deuxième jet par concentration des eaux-mères. Pds: 298 mg; F: 252°C.

Puis on évapore à sec les eaux-mères, les reprend par 200 ml de chlorure de méthylène, lave avec 4 fois 100 ml d'eau, et extrait par 4 fois 250 ml d'HCL N. On alcalinise les phases acides jusqu'à pH 9 et extrait 300 ml de chlorure de méthylène. On sèche sur sulfate de sodium, évapore sous vide, et chromatographie sur 150 ml de silice. Eluant: éther/chloroforme: 1/2 puis éther/chloroforme/acétone: 2/4/1.

Pds: 177 mg; F: 250°C (éthylénique trans)

Pds: 630 mg; F: 143°C (éthylénique cis)

Rdt global: 70%.

Etape f. Méthoxy-10,7-H-Pyrido[4,3-c]carbazole

R = $OCH_3$; R' = R'' = H.

Une solution de 1,2 g de 1-(2-indolyl)-2-(4-pyridyléthylène) cis et trans et 250 mg d'iode dans un litre d'éthanol absolu est irradiée 90 heures dans un photoréacteur de marque «Rayonet» 3500 Å. On concentre l'éthanol à chaud sous vide restreint et laisse cristalliser.

Pds: 757 mg; F: 280°C.

Les eaux-mères sont traitées par 200 ml d'eau, puis 2,3 g de carbonate de sodium en solution dans 20 ml d'eau et 900 mg de thiosulfate de sodium en solution dans 20 ml d'eau. On évapore sous vide l'éthanol et extrait par 100 ml de chloroforme. On essore le produit précipité, le lave par 5 fois 20 ml d'eau.

Pds: 167 mg; F: 285°C.

Le solide obtenu est recristallisé dans le DMF.

Rdt: 75%; F: 288°C.

Exemple 2

Synthèse du méthoxy-10, méthyl-7-pyrido[4,3-c]-carbazole, dérivé 3

R = $OCH_3$, R' = $CH_3$, R'' = H.

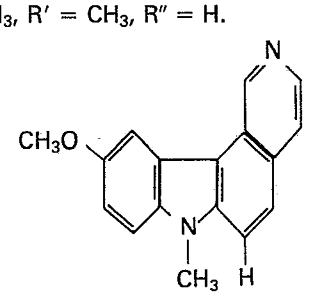

On dissout 1,20 mg de méthoxy-10 7-H-pyrido[4,3-c]carbazole dans 5 ml de DMF. Puis on ajoute 210 mg d'hydrure de sodium 50% en dispersion dans l'huile. On ajoute goutte à goutte 0,13 ml d'iodure de méthyle dilué dans 2 ml de DMF. On laisse une nuit sous agitation à température ambiante, évapore le DMF et chromatographie sur silice de marque MERCK, 100 ml de silice, éluant: chloroforme/méthanol: 9/1.

Pds: 290 mg; F: 129°C; Rdt: 69%.

Exemple 3

Synthèse du hydroxy-10, 7-H-pyrido[4,3-c]carbazole, dérivé 5

R = OH; R' = H; R'' = H.

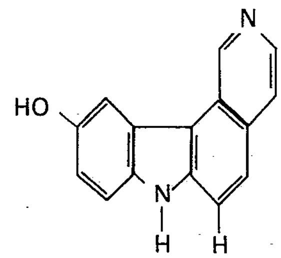

On mélange 515 mg de méthoxy-10 7-H-pyrido[4,3-c]-carbazole et 4 ml d'acide bromhydrique, 48% aqueux incolore. La suspension est dissoute par chauffage. On chauffe à 110–120°C pendant 5 heures, sous azote et agitation. On verse ensuite la solution tiède dans 150 ml d'eau. On traite par une solution de $Na_2CO_3$ aqueuse saturée jusqu'à absence de précipitation (pH = 9). On essore le précipité, recristallise sans sécher dans le méthanol, après passage au noir.

Pds: 220 mg; F: 290°C; Rdt: 45%.

Exemple 4

Synthèse du méthyl-7, hydroxy-10, 7-H-pyrido-[4,3-c]carbazole, dérivé 6

R = OH; R' = $CH_3$; R'' = H.

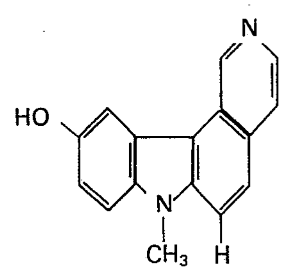

6,3 g de méthyl-7, méthoxy-10, 7-H-pyrido[4,3-c]carbazole sont mélangés à 120 g de chlorhydrate de pyridinium et le mélange est chauffé 2 heures à 210°C. On refroidit et dilue par 300 ml de solution aqueuse saturée de NaCl. On filtre le solide obtenu, lave avec NaCl saturée aqueuse. On dissout dans 500 ml d'eau chaude, laisse revenir à température ambiante, et verse dans 250 ml de solution de $NaHCO_3$ 5%. On filtre et on sèche sous vide le précipité obtenu. On refroidit et obtient ainsi 5 g de méthyl-7, hydroxy-10, 7-H-pyrido[4,3-c]-carbazole.

F: 290°C; Rdt: 74%.

Exemple 5

Synthèse du cyano-6, méthoxy-10, 7-H-pyrido[4,3-c]carbazole, dérivé 9

R = $OCH_3$; R' = H; R'' = CN.

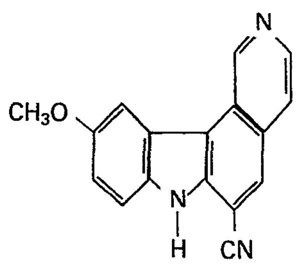

Etape a. Méthoxy-5, cyanométhyl-2 indole

Cette synthèse est calquée sur le procédé décrit par W. Schindler, Helv. Chim. Acta (1975) p. 2158, dans le cas du cyanométhyl-2 indole.

Dans un tricol de 100 ml, sous agitation et sous atmosphère d'azote, on introduit 17,17 g d'iodure de triméthyl-ammoniométhyl-2 méthoxy-5 indole dissout dans 250 ml de méthanol anhydre, puis en une seule fois 11,5 g de KCN pur dissout dans 250 ml de méthanol anhydre.

On laisse agiter 18 heures au reflux. On concentre sous vide le méthanol jusqu'à un volume de 100 ml et on verse dans l'eau, extrait trois fois avec 100 ml d'éther. Les fractions éthérées sont lavées par deux fois 100 ml d'eau, séchées, sur sulfate de sodium, filtrées sur célite et charbon animal, évaporées sous vide. On obtient des cristaux gris.

Pds: 6,36 g; Rdt: 70%; F: > 290°C.

Etape b. 1-cyano, 1'-[(2-méthoxy-5)indole]-2-(4-pyridil)éthylène cis et trans

On dissout par léger chauffage 3,61 g du dérivé précédent dans 125 ml de méthanol et on ajoute simultanément par deux ampoules à brome, en 10 mn à 25°C, une solution de 600 mg de Na dans 50 ml de $CH_3OH$ et une solution de 2,1 ml de formyl-4 pyridine dans 30 ml de $CH_3OH$. On laisse agiter une heure à 25°C. On essore les cristaux formés.

Pds: 3,24 g.

Par évaporation de la solution et reprise par le benzène, on obtient à nouveau 0,256 g. Rdt total: 72%; F: 179°C.

Etape c. Cyano-6, méthoxy-10, 7-H-pyrido[4,3-c]carbazole, dérivé 9

1,7 g du dérivé précédent sont mis en solution dans 1,5 l d'éthanol absolu. On ajoute 300 mg d'$I_2$ bisublimé. Le mélange est irradié 30 heures dans un photoréacteur avec une lampe UV de marque «Hanau TQ 150» haute pression. On recueille les cristaux formés.

Pds: 0,855 g; Rdt: 51%; F: > 290°C.

Exemple 6

Synthèse du carboxamido-6, méthoxy-10, 7-H-pyrido[4,3-c]carbazole, dérivé 11

$R = OCH_3$, $R' = H$; $R'' = CONH_2$.

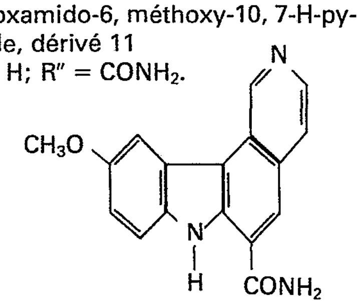

A une suspension de 100 mg de cyano-6, méthoxy-10, 7-H-pyrido[4,3-c]carbazole (dérivé 9) dans 3 ml de DMF, on ajoute 0,3 ml d'eau oxygénée à 110 volumes diluée dans 5 ml d'éthanol, puis 0,2 ml de soude 6N. Tout devient soluble.

On laisse 3 heures à 50°C–60°C, laisse refroidir et essore le solide obtenu. Celui-ci est lavé trois fois avec 10 ml d'eau, séché sous vide.

Pds: 100 mg; F: > 290°C; Rdt: 94%.

Exemple 7

Synthèse du carboxy-6, methoxy-10, 7-H-pyrido[4,3-c]carbazole, dérivé 12

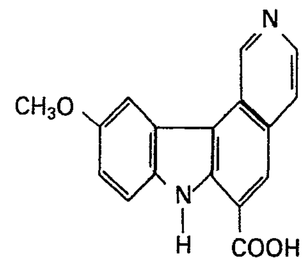

On chauffe 12 heures en tube scellé à 150°C un mélange de 1 g de cyano-6, méthoxy-10, 7-H-pyrido[4,3-c]carbazole (dérivé 9) et 25 ml d'acide chlorhydrique concentré. On évapore à sec le mélange, redissout le solide dans l'eau et neutralise par l'ammoniaque. On essore le précipité et sèche sous vide.

Pds: 580 mg; F: > 290°C; Rdt: 59%.

Exemple 8

Synthèse de l'aminométhyl-6, méthoxy-10, 7-H-pyrido[4,3-c]-carbazole, dérivé 17

$R = OCH_3$; $R' = H$; $R'' = CH_2NH_2$.

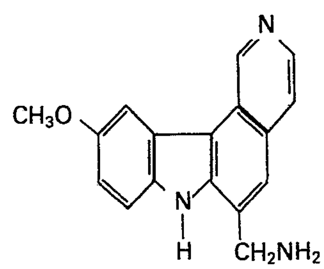

On verse 2 g de cyano-6, méthoxy-10, 7-H-pyrido[4,3-c]carbazole et 400 mg de nickel de Raney dans 100 ml d'alcool isopropylique. Puis on laisse 15 minutes sous ammoniaque (5 atmosphères) et 2 heures à 80°C sous 80 atmosphères d'hydrogène. On évapore sous vide jusqu'à environ 40 ml et acidifie par 10 ml d'acide chlorhydrique N.

On essore le solide obtenu, puis le met en solution dans l'eau, basifie par $Na_2CO_3$ et extrait au chlorure de méthylène. On évapore sous vide, et cristallise dans l'éthanol.

Pds: 1,2 g; f: > 290°C; Rdt: 59%.

Exemple 9

Synthèse du nitro-10, 7-H-pyrido[4,3-c]carbazole, dérivé 15

$R = NO_2$; $R' = H$; $R'' = H$.

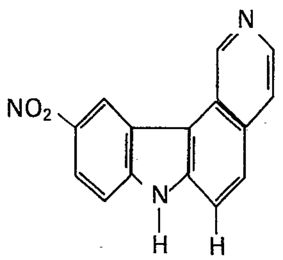

2 g de 7-H-pyrido[4,3-c]carbazole 1 équivalent (1 éq.) sont dissous dans 20 ml d'$H_2SO_4$, Cl = 1,84. On verse 950 mg de nitrate de potassium (1,02 éq.). On laisse deux heures sous agitation, puis verse dans 50 ml de glace, alcalinise par NaOH. On essore le solide obtenu et sèche sous vide.

Pds: 1,65 g; F: > 290°C; Rdt: 68%.

Exemple 10

Synthèse de l'ester propionique de l'hydroxy-10, 7-H-pyrido[4,3-c]-carbazole, dérivé 13

R = $OCOCH_2CH_3$; R' = H; R" = H.

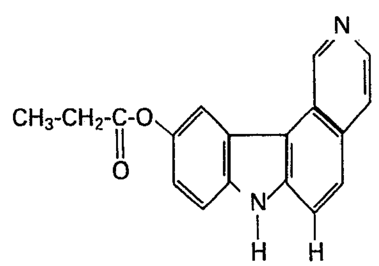

A une suspension de 1,3 g d'hydroxy-10, 7-H-pyrido[4,3-c]carbazole (1 éq.) et 5 g de carbonate de sodium, dans 75 ml d'acétone, on ajoute 770 mg (1,5 éq.) de chlorure de propionyle. Après deux heures, on ajoute lentement 500 ml d'eau. On recueille le précipité, lave avec trois fois 50 ml d'eau chaude.

Le solide est séché sous vide et recristallisé dans un mélange chloroforme/éthanol.

Pds: 970 mg; F: 213°C; Rdt: 60%.

Exemple 11

Synthèse du propyloxy-10, 7-H-pyrido[4,3-c]carbazole, dérivé 14

R = $O(CH_2)_2CH_3$; R' = H; R" = H.

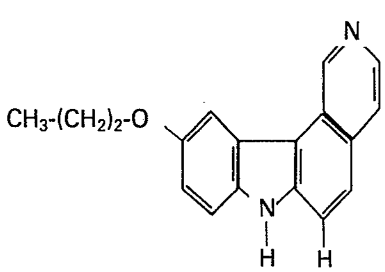

On chauffe au reflux pendant 48 heures un mélange de 2 g de hydroxy-10, 7-H-pyrido[4,3-c]carbazole, 2 ml de N,N-diméthyl formamide dinéopentyl acétal et 40 ml de propanol. On évapore le solvant et reprend le résidu par 700 ml d'acétate d'éthyle. On concentre la solution, après passage sur charbon jusqu'à 200 ml environ. On essore le solide obtenu et sèche sous vide.

Pds: 1,3 g; F: 280°C; Rdt: 53%.

On a préparé par ces méthodes générales les dérivés des 7-H-pyrido[4,3-c]carbazoles, rassemblés dans le tableau I, des 7-H-pyrido[3,4-c]carbazoles, rassemblés dans le tableau II et des 7-H-pyrido[2,3-c]carbazoles, rassemblés dans le tableau III.

Tableau I

Dérivés du 7-H-pyrido[4,3-c]carbazole

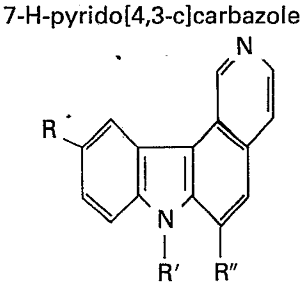

| Dérivé | R | R' | R" | PM | F (°C) |
|---|---|---|---|---|---|
| 1 | $OCH_3$ | H | H | 248 | 283 |
| 2 | H | H | H | 218 | 259 |
| 3 | $OCH_3$ | $CH_3$ | H | 262 | 129 |
| 4 | H | $CH_3$ | H | 232 | 117 |
| 5 | OH | H | H | 234 | 360 |
| 6 | OH | $CH_3$ | H | 248 | 183 |
| 7 | Br | H | H | 297 | 280 |
| 8 | $OCOCH_3$ | H | H | 276 | 225 |
| 9 | $OCH_3$ | H | CN | 273 | >290 |
| 10 | $OCH_3$ | $CH_3$ | CN | 287 | >290 |
| 11 | $OCH_3$ | H | $CONH_2$ | 305 | >290 |
| 12 | $OCH_3$ | H | COOH | 298 | >290 |
| 13 | $OCOCH_2CH_3$ | H | H | 290 | 213 |
| 14 | $O(CH_2)_2CH_3$ | H | H | 287 | 280 |
| 15 | $NO_2$ | H | H | 263 | >290 |
| 16 | $NH_2$ | H | H | 233 | >290 |
| 17 | $OCH_3$ | H | $CH_2NH_2$ | 277 | >290 |

Tableau II

Dérivés du 7-H-pyrido[3,4-c]carbazole

R" = H

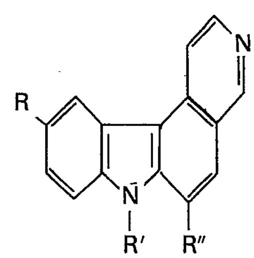

| Dérivé | R | R' | PM | F (°C) |
|---|---|---|---|---|
| 18 | $OCH_3$ | H | 248 | 254 |
| 19 | H | H | 218 | 253 |
| 20 | $OCH_3$ | $CH_3$ | 262 | 156 |
| 21 | H | $CH_3$ | 232 | 130 |
| 22 | OH | H | 234 | 352 |
| 23 | OH | $CH_3$ | 248 | 170 |
| 24 | Br | H | 297 | 250 |
| 25 | $OCOCH_3$ | H | 276 | 217 |

Tableau III
Dérivés du 7-H-pyrido[2,3-c]carbazole
R″ = H

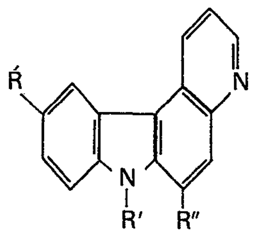

| Dérivé | R | R′ | PM | F (°C) |
|---|---|---|---|---|
| 25 | $OCH_3$ | H | 248 | 232 |
| 27 | H | H | 218 | 212 |
| 28 | $OCH_3$ | $CH_3$ | 262 | 160 |
| 29 | H | $CH_3$ | 232 | 152 |
| 30 | OH | H | 234 | 270 |
| 31 | OH | $CH_3$ | 248 | 164 |
| 32 | Br | H | 297 | 260 |
| 33 | $OCOCH_3$ | H | 276 | 215 |

Synthèse des dérivés de bis 7 H pyridocarbazoles

Exemple 12
Dichlorure de 1′1′-bis 2-(2-méthoxy-10, 7-H-pyrido[4,3-c]carbazolium)éthyl14,4′ bispipéridine, dérivé 34

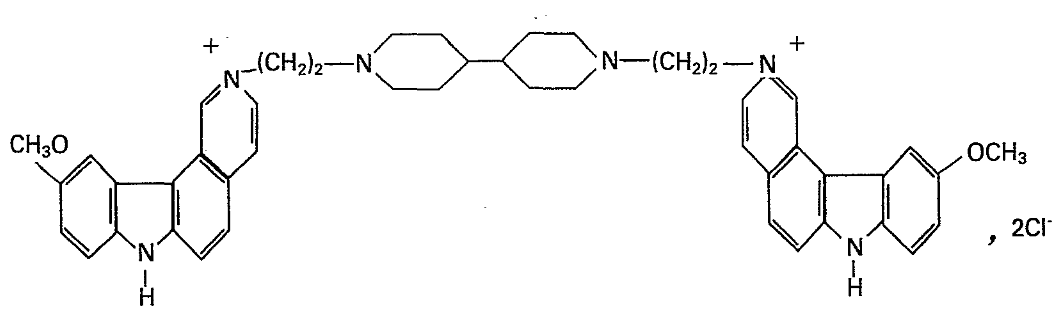

On dissout 100 mg (2 éq.) de méthoxy-10, 7-H-pyrido[4,3-c]carbazole dans 3 ml de DMF. On ajoute 70 mg (1,2 éq.) de 1,1′bis (2-chloroéthyl) 4,4′ bispipéridine dans 1 ml de DMF. On laisse 16 heures à 100°C sous agitation, puis 4 heures à température ambiante. On essore le solide, lave avec 5 fois 5 ml d'éther éthylique. On sèche sous vide.

Pds: 95 mg; Rdt: 60%; F: 292°C.

Exemple 13
Dichlorure de N,N′ bis 2-(2-méthoxy-10, 7-H-pyrido[4,3-c]carbazolium)éthyl pipérazine, dérivé 35

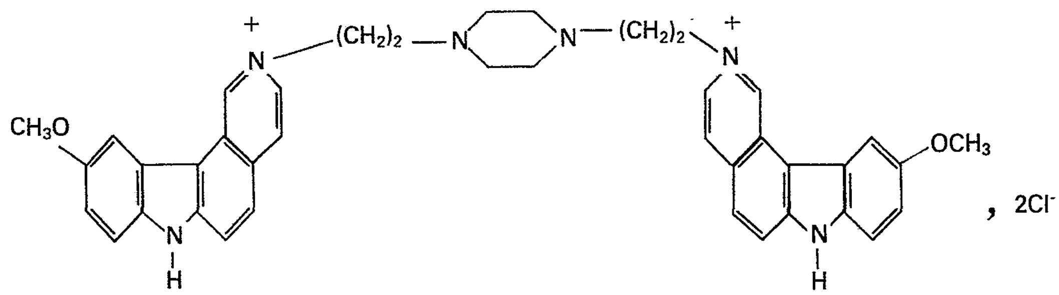

On dissout 100 mg (2 éq.) de méthoxy-10 7-H-pyrido[4,3-c]carbazole dans 3 ml de DMF à 100°C. On ajoute une solution de 50 mg (1,2 éq.) de N,N′ bis chloroéthyl pipérazine dans 1 ml de DMF. On laisse 16 heures à 100°C sous agitation puis 4 heures à température ambiante. On essore le solide, lave avec 5 fois 5 ml d'éther éthylique et sèche sous vide.

Exemple 14
Dichlorure de 2,3 bis N-(2-(2-méthoxy-10, 7-H-pyrido[4,3-c]carbazolium)éthyl) 4-pipéridyl propane, dérivé 36

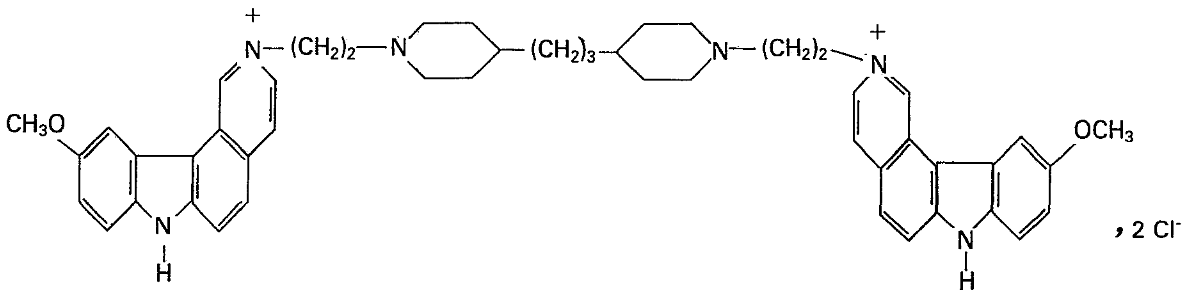

On dissout 100 mg (2 éq.) de méthoxy-10, 7-H-pyrido[4,3-c]carbazole dans 3 ml de DMF à 100°C. On ajoute une solution de 80 mg de 1,3 bis N-(2-chloroéthyl) 4-pipéridyl propane dans 1 ml de DMF. On laisse 16 heures à 100°C sous agitation puis 4 h à température ambiante. On essore le solide, lave avec 5 fois 5 ml d'éther éthylique. On sèche sous vide.

Pds: 90 mg; Rdt: 67%; F: 290°C.

Exemple 15

Dichlorure de diaza 3–10 dodécaméthylène-2,2' bis méthoxy-10, 7-H-pyrido[4,3-c]carbazolium, dérivé 37

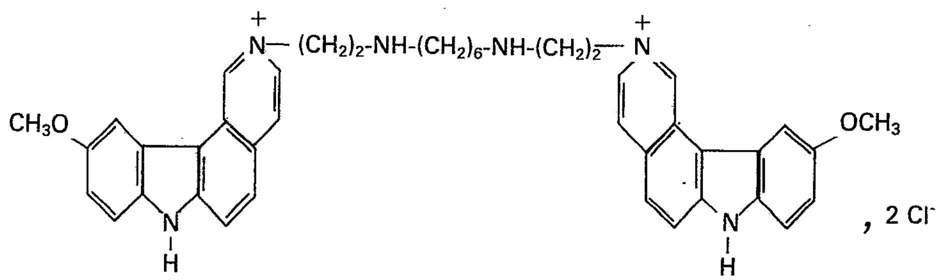

On dissout 100 mg (2 éq.) de méthoxy-10, 7-H-pyrido[4,3-c]carbazole dans 3 ml de DMF à 100°C. On dissout d'autre part 70 mg (1,7 éq.) de 1,6 di(2-chloroéthylamino)hexane dichlorydrate dans 6 ml d'eau, basifie par 0,05 ml de soude 10N en présence de chlorure de méthylène (3 ml). On extrait avec 5 ml de chlorure de méthylène, sèche la phase organique sur -$CaCl_2$, filtre et verse dans la solution de DMF chaude. On laisse le chlorure de méthylène s'évaporer et laisse une nuit à 100°C. On essore, sèche sous vide.

Pds: 74 mg; Rdt: 50%; F: 290°C.

Exemple 16

1,6 bis(2-(2-méthoxy-10, 7-H-pyrido[4,3-c]carbazolium éthylamino)-1,6-didéoxy-D-mannitol, dérivé 39

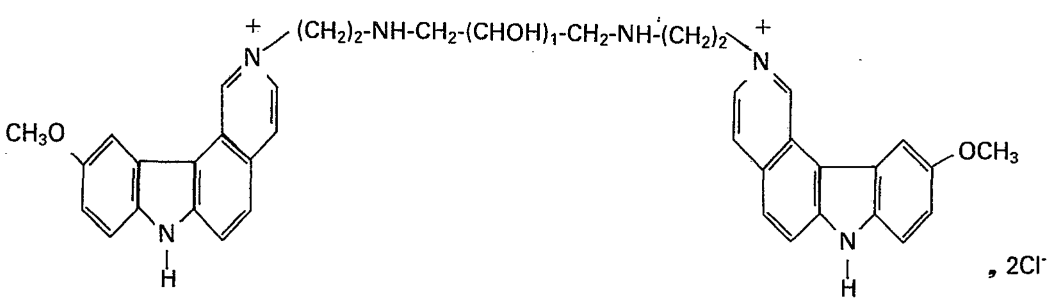

On dissout 100 mg (2 éq.) de méthoxy-10, 7-H-pyrido[4,3-c]carbazole dans 3 ml de DMF à 60°C. On ajoute ensuite 70 mg de 1,6 bis(2-chloroéthylamino)-1,6 didéoxy-D-mannitol (1,15 éq.). On laisse une nuit à 100°C, puis essore, sèche sous vide.

Pds: 90 mg; F: 290°C; Rdt: 52%.

Exemple 17

Dichlorure de 1,1' bis 2-(2-hydroxy-10, 7-H-pyrido[4,3-c]carbazolium)éthyl 4,4')bispipéridine, dérivé 40

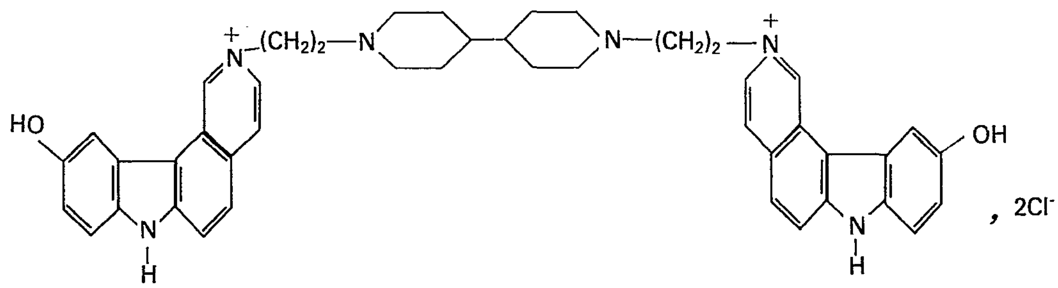

On dissout 100 mg (2 éq.) d'hydroxy-10, 7-H-pyrido[4,3-c]carbazole dans 3 ml de DMF à 100°C. On ajoute 70 mg (1,15 éq.) de 1,1' bis (2-chloroéthyl) 4,4' bispipéridine dilués dans 1 ml de DMF. On laisse 16 heures à 100°C sous agitation, puis 4 heures à température ambiante. On essore et sèche le solide obtenu.

Pds: 105 mg; F: 290°C; Rdt: 66%.

Exemple 18

Dichlorure de 1,1' bis 2-(2-méthyl-7-méthoxy-10, 7-H-pyrido[4,3-c]carbazolium) éthyl 4,4' bispipéridine, dérivé 41

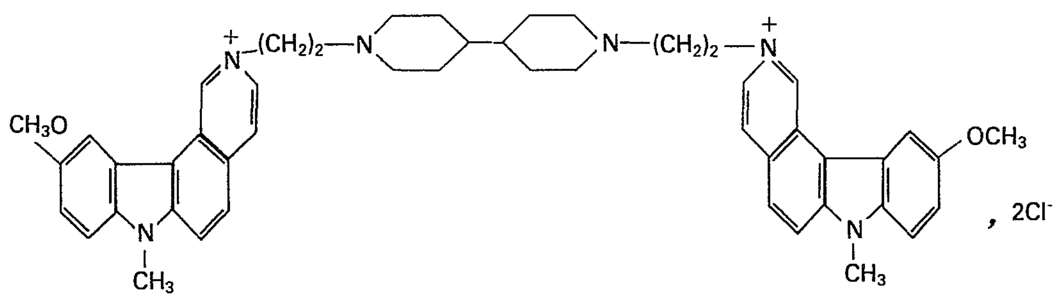

On dissout 100 mg (2 éq.) de méthyl-7, méthoxy-10, 7-H-pyrido[4,3-c]carbazole dans 3 ml de DMF à 95–100°C. Puis on ajoute 67 mg (1,2 éq.) de 1,1' (2-chloroéthyl) 4,4' bispipéridine dilués dans 1 ml de DMF. On laisse 16 heures à 95–100°C sous agitation, puis 4 heures à température ambiante. On essore le solide obtenu, rince deux fois avec 5 ml d'éther, sèche le solide obtenu.

Pds: 86 mg; F: 250°C; Rdt: 55%.

Exemple 19

Dichlorure de 1,1'bis 2-hydroxy-3-(2-méthoxy-10, 7-H-pyrido[4,3-c]carbazolium)propyl 4,4' bispipéridine, dérivé 42

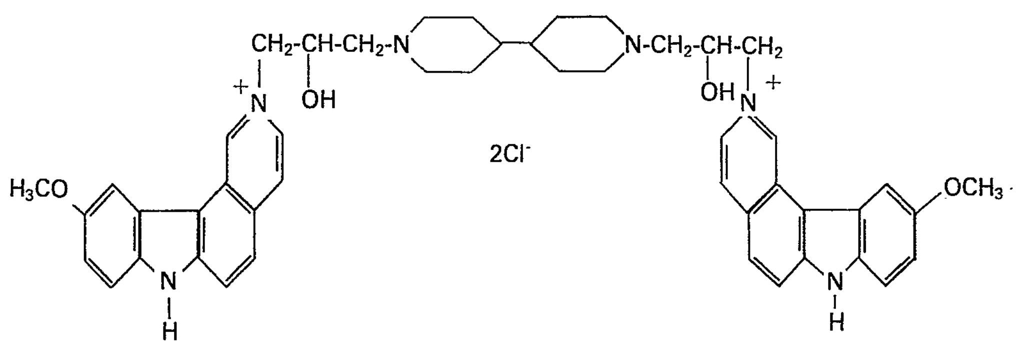

On dissout 100 mg (2 éq.) de méthoxy-10, 7-H-pyrido[4,3-c]carbazole dans 3 ml de DMF à 105°C. On ajoute 85 mg (1 éq.) de 1,1'bis(2-hydroxy-3-chloropropyl)4,4)bispipéridine dilués dans 1 ml de DMF et 5 ml de méthanol. On laisse 16 heures à 105°C sous agitation, concentre sous vide jusqu'à 2 ml et refroidit à 5–10°C. On ajoute ensuite 2 ml d'acétone et essore le précipité obtenu (68 mg).

Ce solide est chromatographié sur une colonne de résine connue sous la désignation Servachrom XAD-2 dans un mélange de méthanol et de tampon acétate d'ammonium, 0,1 M, à pH 6, et dans le rapport 30/70.

Exemple 20

Dichlorure de N,N'diméthyl diaza3–9 nona méthylène-2-2' bis méthoxy-10, 7-H-pyrido[4,3-c]carbazolium, dérivé 43

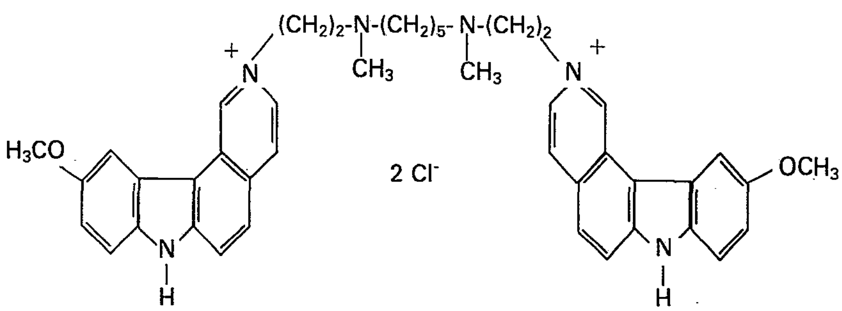

On dissout 100 mg (2 éq.) de méthoxy-10, 7-H-pyrido[4,3-c]carbazole dans 3 ml de DMF à 85°C. On dissout d'autre part 60 mg (1 éq.) de N,N' diméthyl 1,6 di(2-chloroéthylamino) pentane dichlorhydrate dans 5 ml d'eau, basifie par 0,2 ml de soude 10N en présence de chloroforme (5 ml). La phase organique est séchée sur $CaCl_2$, filtrée et versée dans la solution de DMF chaude. On laisse une nuit à 85°C, le DMF est évaporé sous vide et le résidu (49 mg) est chromatographié sur une colonne de résine connue sous la désignation Servachrom-XAD-2 dans un mélange de méthanol et de tempon acétate d'ammonium 0,1 M à pH 6 et dans le rapport 50/60.

Pds: 32 mg; F: >290°C; Rdt: 25%.

Exemple 21

Dibromure de 1,10 bis (2-méthoxy-10, 7-H-pyrido[4,3-c]carbazolium) décane, dérivé 44

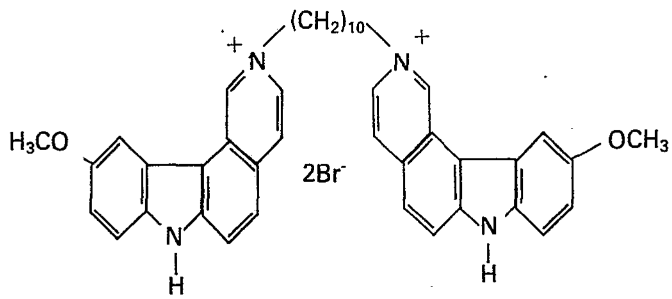

On dissout 100 mg (2 éq.) de méthoxy-10, 7-H-pyrido[4,3-c]carbazole dans 3 ml de DMF à 10°C, puis on ajoute 45 mg (1 éq.) de dibromodécane dilué dans 1 ml de DMF. On laisse 16 heures sous agitation à 100°C puis 4 heures à température ambiante. On essore le solide, lave avec 5 fois 5 ml d'éther éthylique. On sèche sous vide.

Pds: 66 mg; F: >290°C; Rdt: 47%.

Exemple 22

Dichlorure de 1,1'-bis 2-(3-méthoxy-10, 7-H-pyrido[3,2-c]carbazolium)éthyl 4,4' bispipéridine, dérivé 45

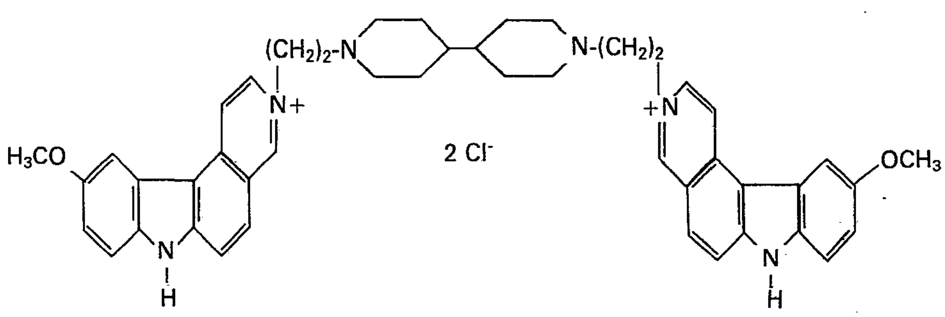

On dissout 100 mg (2 éq.) de méthoxy-10, 7-H-pyrido[3,2-c]carbazole dans 3 ml de DMF. On ajoute 70 mg (1,2 éq.) de 1,1' bis (2)chloroéthyl) 4,4' bispipéridine dans 1 ml de DMF. On laisse 16 heures à 100°C sous agitation puis 4 heures à température ambiante. On essore le solide, lave avec 5 fois 5 ml d'éther éthylique. On sèche sous vide.

Pds: 90 mg; F: 280°C; Rdt: 57%.

Exemple 23

Dichlorure de 1,1' bis 2-(3-hydroxy-10, 7-H-pyrido[3,2-c]carbazolium) éthyl 4,4' bispipéridine, dérivé 46

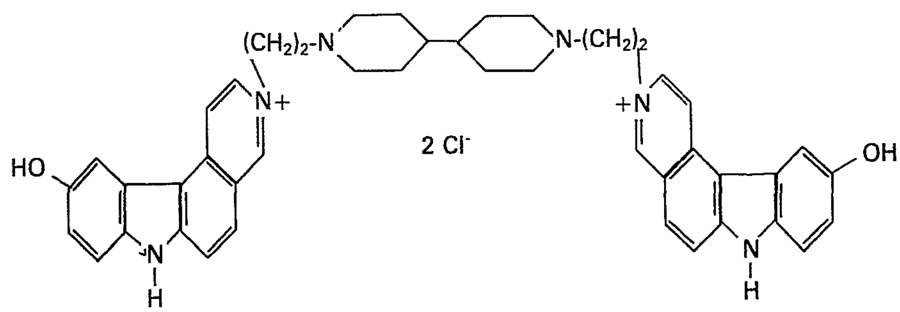

On dissout 100 mg (2 éq.) d'hydroxy-10, 7-H-pyrido[3,2-c]carbazole dans 3 ml de DMF à 100°C. On ajoute 70 mg (1,15 éq.) de 1,1' bis (2-chloroéthyl) 4,4' bispipéridine dilués dans 1 ml de DMF. On laisse 16 heures à 100°C, puis 4 heures à température ambiante. On essore et sèche le solide obtenu.

Pds: 100 mg; F: 260°C; Rdt: 69%.

Exemple 24

Dichlorure de 1,1' bis 2-(3-méthyl-7-méthoxy-10, 7-H-pyrido[3,2-c]carbazolium) éthyl 4,4' bispipéridine, dérivé 47

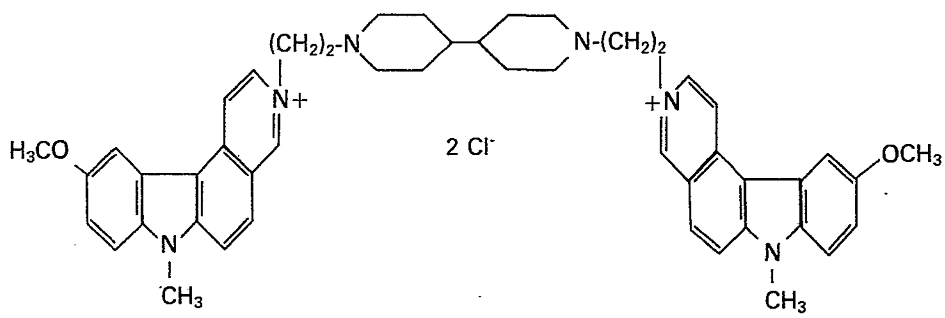

On dissout 100 mg (2 éq.) de méthyl-7 méthoxy-10, 7-H-pyrido[3,2-c]carbazole dans 3 ml de DMF à 95–100°C, puis on ajoute 67 mg (1,2 éq.) de 1,1' bis (2-chloroéthyl) 4,4' bispipéridine dilués dans 1 ml de DMF. On laisse 16 heures à 95–100°C sous agitation, puis 4 heures à température ambiante. On essore le solide obtenu, rince avec 2 fois 5 ml d'éther, sèche sous vide.

Pds: 87 mg; F: >290°C; Rdt: 56%.

Exemple 25

Dichlorure de 1,1' bis 2-(4-méthyl-7, 7-H-pyrido[2,3-c]carbazolium) éthyl 4,4' bispipéridine, dérivé 48

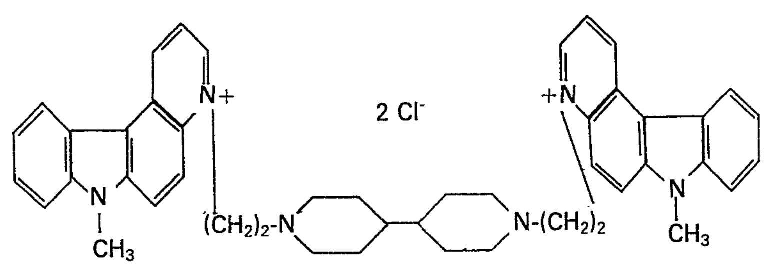

On dissout 100 mg (2 éq.) de méthyl-7, 7-H-pyrido[2,3-c]carbazole dans 3 ml de DMF à 100°C. On ajoute 70 mg (1,1 éq.) de 1,1' bis (2-chloroéthyl) 4,4' bispipéridine dilués dans 1 ml de DMF. On laisse 16 heures à 100°C sous agitation, puis 4 heures à température ambiante. On essore et sèche le solide obtenu.

Pds: 28 mg; F: >290°C; Rdt: 17%.

Tous les composés selon la présente invention sont caractérisés par leur point de fusion; leur structure est confirmée par RMN; leur microanalyse est conforme à la théorie.

Selon les techniques décrites dans les exemples précédents correspondants, on prépare tous les groupes 7 H pyridocarbazoles rassemblés dans les tableaux I, II et III. Ces groupes sont dimérisés par l'intermédiaire de chaînes Z de formule $-(CH_2)_n-X-Y-X'-(CH_2)_{n'}$, dans lesquelles les groupements X et X' sont identiques et les nombres n et n' sont égaux. Des exemples de chaînes sont rassemblés au tableau IV.

Tableau IV

| X | Y | n |
|---|---|---|
| pipéridine | – | 2 |
| pipéridine | – | 3 |
| pipéridine | $(CH_2)_2$ | 2 |
| pipéridine | $(CH_2)_2$ | 3 |

| X | Y | n |
|---|---|---|
| – | $(CH_2)_3$ | 2 |
| – | $(CH_2)_3$ | 3 |
| – | pipérazine | 2 |
| – | – | 3 |
| NH | $(CH_2)_4$ | 2 |
| – | $(CH_2)_5$ | 2 |
| – | $(CH_2)_6$ | 2 |
| NH | $(CH_2)_4$ | 3 |
| NH | $CH_2$-$(CHOH)_4$-$CH_2$ | 2 |

Tous les groupes 7 H pyridocarbazoles des tableaux I, II et III sont dimérisés selon les modes de dimérisation précédemment décrits.

Le tableau V rassemble quelques composés particuliers.

Tableau V

| Composé | R | R′ | R″ | X = X′ (dans la chaîne-$(CH_2)_n$-X-Y-X′-$(CH_2)_n$) | Y | n = n′ | Position de N dans le cycle pyridyle |
|---|---|---|---|---|---|---|---|
| 34 | $CH_3O$ | H | H | pipéridine | – | 2 | 2 |
| 35 | $CH_3O$ | H | H | – | pipérazine | 2 | 2 |
| 36 | $CH_3O$ | H | H | pipéridine | $(CH_2)_3$ | 2 | 2 |
| 37 | $CH_3O$ | H | H | NH | $(CH_2)_6$ | 2 | 2 |
| 39 | $CH_3O$ | H | H | NH | $CH_2$-$(CHOH)_4$-$CH_2$ | 2 | 2 |
| 40 | OH | H | H | pipéridine | – | 2 | 2 |
| 41 | $CH_3O$ | $CH_3$ | H | pipéridine | – | 2 | 2 |
| 42 | $CH_3O$ | H | H | 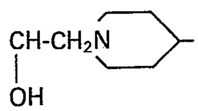 | | 1 | 2 |
| 43 | $CH_3O$ | H | H | -N-<br>$CH_3$ | $(CH_2)_5$ | 2 | 2 |
| 44 | $CH_3O$ | H | H | – | $(CH_2)_2$ | 4 | 2 |
| 45 | $CH_3O$ | H | H | pipéridine | – | 2 | 3 |
| 46 | HO | H | H | pipéridine | – | 2 | 3 |
| 47 | $CH_3O$ | $CH_3$ | H | pipéridine | – | 2 | 3 |
| 48 | H | $CH_3$ | H | pipéridine | – | 2 | 4 |

Etude pharmacologique

Afin de tester l'activité antitumorale des composés décrites plus haut, on a utilisé en premier lieu des leucémies murines P388 et L1210. La leucémie P388 est considérée comme très sensible et permet une première sélection. La leucémie L1210 est plus résistante et plus sélective. Une substance ayant une grande activité sur la leucémie L1210 a une grande chance d'être active en clinique humaine (J. M. Venditti, Relevance of transplantable animal tumor systems to the Selection of new agents for clinical trial in pharmacological basis of cancer chemotherapy. The University of Texas ed. Williams and Wilkins, publ. 1975, Baltimore USA).

D'autre part, la tumeur expérimentale qu'est la leucémie L1210 de la souris est en effet couramment utilisée pour l'évaluation de tous les composés antitumoraux actuellement mis en œuvre en thérapeutique humaine, ainsi qu'il est décrit, par exemple par C. C. Zubrod dans Proc. Nat. Acad. Sci. USA 69, 1972, p. 1042–1047. Le système tumoral ainsi constitué expérimentalement permet une évaluation quantitative très précise de l'activité du composé testé, et, par conséquent aussi, une comparaison objective entre les activités respectives de différents composés, par exemple selon les méthodes décrites par R. E. Skipper, F. M. Schabel, Jr. et W. S. Wilcox dans Cancer Chemother. Rep., 35, 1964, p. 1–111 et 45, 1965, p. 5–28.

En pratique, on a utilisé des souris ayant reçu par voie intrapéritonéale un inoculum de cellules de leucémie L1210 et ayant été, pour la moitié d'entre elles, soumises 24 heures après ladite inoculation tumorale à une injection intrapéritonéale d'une dose unique de l'un des composés à tester; l'autre moitié des souris à reçu une injection d'un volume identique d'un solvant inactif et servait de série témoin. On a réparti les souris au hasard dans chaque série expérimentale. Une première expérimentation de routine a permis de déterminer la mortalité par toxicité et de fixer ainsi les doses infra-létales.

Conformément à la convention en usage dans ce domaine, on considérait comme guéris les animaux qui survivaient plus de 45 jours après l'inoculation de cellules tumorales.

On a ainsi pu déterminer la mortalité par toxicité sous l'effet d'une injection unique de l'un des composés à différentes doses (doses infra-létales, DL 50 et DL 100) et évaluer le taux de survie du groupe d'animaux auxquels on n'avait injecté qu'une dose infra-létale d'un composé selon l'invention.

On a calculé le taux de survie à partir d'une analyse statistique des résultats expérimentaux, par comparaison avec les résultats obtenus avec la série témoin, selon les méthodes bien connues de Mann-Whitey et de Wilcoxon; le pourcentage de cellules leucémiques tuées par le traitement réalisé était calculé par la méthode décrite par H. E. Skipper et coll. dans les articles cités plus haut, en prenant pour base, suivant le cas, soit l'augmentation de la survie moyenne en l'absence de survivants, soit le pourcentage de survivants.

Pour l'évaluation de l'activité thérapeutique des composés selon l'invention, on a exprimé les doses dudit composé en les rapportant à la dose infra-létale 30 jours, que l'on a posée égale à 1. Cette dose infra-létale s'est en effet avérée plus facile à déterminer pour les composés de l'invention que la DL 10, dont elle est d'ailleurs très voisine et qui est habituellement prise comme référence pour l'évaluation de l'activité thérapeutique de composés à effet antitumoral (voir H. E. Skipper et coll., ouvrages cités).

L'activité thérapeutique sur la leucémie P388 de la souris est comparable à celle obtenue sur la leucémie L1210 pour les mêmes doses, le protocole étant identique.

En outre, ces composés présentent une forte activité sur le mélanome B 16 et la tumeur de Lewis, pour les mêmes doses, le protocole étant identique. A titre d'exemple, sur le mélanome B 16, le dérivé 41 augmente de 15 jours la survie des souris traitées; sur la tumeur de Lewis, l'augmentation de survie induite pour ce même dérivé est de 8 jours avec un survivant.

L'ensemble des substances dont la synthèse est décrite dans la première partie a été testé sur ces systèmes. A partir des résultats obtenus, les conclusions suivantes peuvent être tirées:

1° les molécules de 7 H pyridocarbazoles ont une activité antitumorale faible mais significative;

2° certaines molécules de bis 7 H pyridocarbazoles ont une activité antitumorale très élevée et l'activité est reliée aux facteurs suivants:

position de départ de la chaîne reliant les deux noyaux. La série des 7-H-pyrido[4,3-c]carbazolium est la plus active;

nature et conformation de la chaîne reliant les deux noyaux. Les molécules ayant une chaîne semi-rigide sont apparues plus actives.

Un exemple des activités obtenues est donné sur le tableau VI.

Tableau VI

| Dose en fraction de la dose infra-létale | Survie moyenne en jours des souris témoins ± erreur standard | Survie moyenne en jours des souris traitées ± erreur standard | % de cellules tumorales tuées par le traitement | Augmentation survie traitées/contrôle × 100 |
|---|---|---|---|---|
| Dérivé 34 | | | | |
| 0.02 | 9.9 ± 1 | 12.2 ± 0.9 | 98 | 128 |
| 0.1 | 9.9 ± 1 | 13.6 ± 2.9 | 94.8 | 137 (1/15) |
| 0.2 | 9,9 ± 1 | 14.3 ± 2.6 | 99.97 | 144 |
| 0.25 | 8.6 ± 0.7 | 18.6 ± 4.5 | 99.999 | 216 |
| 0.3 | 9.2 ± 0.9 | 17.6 ± 4.5 | 99.999 | 191 |
| 0.4 | 9.9 ± 1 | 18.1 ± 4.5 | 99.999 | 182 |
| Dérivé 40 | | | | |
| 0.02 | 7.9 ± 0.7 | 9.4 ± 1.2 | 94 | 111 |
| 0.2 | 8.4 ± 0.6 | 12.9 ± 1.5 | 99.47 | 153 |
| 0.4 | 7.9 ± 0.7 | 13.2 ± 1.9 | 99.994 | 167 |
| Dérivé 41 | | | | |
| 0.04 | 8.3 ± 0.4 | 11.9 ± 1.2 | 99.8 | 143 |
| 0.4 | 8.3 ± 0.4 | 11.8 ± 0.9* | 99.8 | 140 (1/15)* |
| 0.8 | 8.3 ± 0.4 | 12.9 ± 1.9* | 99.97 | 155 (1/15)* |

*Nombre d'animaux guéris

Effet thérapeutique des doses variables (injection unique par voie intrapéritonéale) de différents dérivés dimériques de pyridocarbazoles pour un inoculum tumoral de $10^5$ cellules de leucémie L1210.

A la lecture de ces tableaux, on remarque que: l'index chimiothérapeutique des dérivés 34, 40 et 41 est extrêmement élevé;

pour une dose égale au 1/50 de la dose infra-létale (composés 34 et 40), on a observé une activité encore très grande puisque, selon le cas,

entre 94 et 98% des cellules leucémiques sont tuées. Au 1/15 de la dose infra-létale dans le cas du composé 41, 99,8% des cellules leucémiques sont tuées;

pour une dose qui n'est plus que le 1/5 de la dose infra-létale, l'activité des dérivés 34 et 40 est telle que 99,97% des cellules leucémiques sont tuées.

Aucune substance actuellement connue et utilisée dans la thérapeutique des cancers humains n'a un index chimiothérapeutique aussi favorable (voir J. Venditti et Skipper et coll. cités plus haut).

à une dose égale à environ la moitié de la dose infra-létale, 99,999% des cellules leucémiques sont tuées, soit un chiffre également très élevé.

Pour situer l'activité de ces produits sur la leucémie L1210, le nombre de cellules survivant après le traitement par le dérivé 34 (ce nombre est d'autant plus petit que l'activité thérapeutique est plus élevée) est comparé avec le résultat obtenu avec deux produits les plus actifs connus actuellement sur la L1210; la cyclophosphamide ou endoxan et la biochloréthyl-nitrosourée (BCNU). La figure 2 représente l'activité comparé du dérivé 34, de l'endoxan, et de la BCNU, respectivement représentés par DIE, E et B sur le graphe de la figure 2.

La fraction des cellules survivantes N/No après traitement est portée, en ordonnée, en fonction de la dose utilisée pour le traitement exprimé en fraction de la dose infralétale (DLo) en abscisse.

Il apparaît clairement qu'aussi bien la cyclophosphamide ou endoxan (E) et la bischloréthylnitrosourée (BCNU) (B) ne sont actives qu'à des doses bien supérieures à celles du dérivé 34 (DIE).

Les produits selon l'invention sont donc particulièrement appropriés pour le traitement de divers cancers humains. Notamment ceux qui sont sensibles à une chimiothérapie. Ils sont particulièrement appropriés pour le traitement des diverses formes de cancer répondant à cette condition et qui se trouvent identifiées dans les publications déjà mentionnées.

L'invention concerne aussi les compositions pharmaceutiques comprenant les composés nouveaux susdits en association avec un véhicule pharmaceutique approprié au mode d'administration choisi.

L'invention concerne particulièrement des solutions stériles ou stérilisables, injectables ou propres à être utilisées pour la préparation, notamment extemporence, de solutions injectables aptes à être administrées par injections intradermiques, intramusculaires ou sous-cutanées. Elle concerne en particulier des solutions aqueuses physiologiques, notamment isotoniques, d'un de ces composés telles que des solutions isotoniques salines.

Les doses administrées quotidiennement doivent être suffisantes pour qu'une action puisse se manifester au moins dans une proportion relativement importante de patients atteints de l'une ou l'autre des diverses formes de cancer qui sont ou seront accessibles à la chimiothérapie, cependant sans pour autant excéder celles pour lesquelles les composés deviendraient trop toxiques.

A titre d'exemple, les doses quotidiennes administrées par voie générale, notamment par perfusion, pourront varier d'environ 10 à environ 100 mg par $m^2$ de corps humain.

L'invention concerne également les autres formes d'administration, notamment par voie orale (compositions solides ou liquides) ou par voie rectale (compositions glycérinées appropriées à cette dernière voie).

Du fait de leur activité particulièrement importante, ils sont également utiles comme produits de référence dans des études pharmacologiques, notamment aux fins de comparaison de propriétés antitumorales de produits étudiés par rapport à un produit de référence.

**Revendications**

1. Sel d'ammonium quaternaire d'un dérivé de bis-7 H pyridocarbazole, caractérisé par la formule suivante:

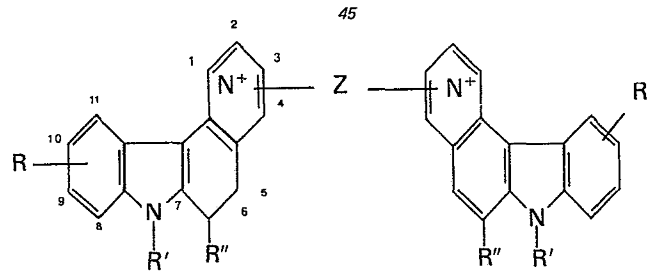

, $2A^-$

dans laquelle:

l'atome d'azote du cycle pyridile occupe l'une des positions 1 à 4; et

Z est une chaîne moléculaire de formule:

$$-(CH_2)_n - W - (CH_2)_{n'}-$$

dans laquelle:

n et n' varient de 2 à 4;

W est choisi parmi les groupes suivants:

$-(CH_2)_m-$

$-NH-(CH_2)_p-(CHOH)_m-(CH_2)_{p'}-NH-$

$-NR_1-(CH_2)_p-(CHOH)_m-(CH_2)_{p'}-NR_1-$

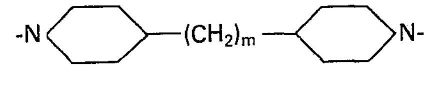

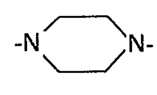

avec m variant de 0 à 4, et p, p' prenant les valeurs 1 ou 2;

-NH-$(CH_2)_{m'}$ -NH-

-$NR_1$-$(CH_2)_{m'}$ -$NR_1$-

avec m' variant de 2 à 8;

le 1,4-diamine benzène,

le groupe 4,4'-diaminobiphényle,

la 4,4'-bipyridine,

le groupe décahydronaphtyridine,

$R_1$ est un groupe alkyle comprenant de 1 à 10 atomes de carbone;

R qui occupe une position quelconque sur le noyau correspondant est un hydrogène, un halogène, un groupe nitro, un groupe amino, le cas échéant substitué par des restes alkyle comprenant de 1 à 10 atomes de carbone, ou des restes acyle comprenant de 2 à 10 atomes de carbone ou de 7 à 10 atomes de carbone lorsque ce sont des restes aroyle, par des restes aryle comprenant de 6 à 10 atomes de carbone, un groupe OH, le cas échéant substitué par des restes alkyle comprenant de 1 à 12 atomes de carbone, acyle comprenant de 2 à 12 atomes de carbone ou de 7 à 12 atomes de carbone lorsque ce sont des restes aroyle, par des restes aryle comprenant de 6 à 12 atomes de carbone;

R' est un hydrogène, un reste alkyle comprenant de 1 à 10 atomes de carbone ou un reste aryle comprenant de 6 à 12 atomes de carbone, ou acyle comprenant de 2 à 10 atomes de carbone ou de 7 à 12 atomes de carbone lorsqu'il s'agit d'un groupe aroyle;

R" est un atome d 'hydrogène,

un radical cyano, formyle, hydroxyméthyle éventuellement estérifié par un acide carboxylique aliphatique de 1 à 10 atomes de carbone,

un radical aminométhyle éventuellement substitué sur l'amino par un groupe alkyle ou acyle de 1 à 10 atomes de carbone,

un radical carboxyle éventuellement estérifié par un alcool aliphatique de 1 à 10 atomes de carbone,

ou un radical amido;

$A^-$ étant l'anion d'un acide physiologiquement acceptable.

2. Sel d'ammonium quaternaire d'un dérivé de bis 7 H pyridocarbazole selon la revendication 1, caractérisé en ce que les atomes d'azote des cycles pyridyle sont en position 2 et en ce que Z est une chaîne de formule:

$$-(CH_2)_n - W - (CH_2)_{n'}-$$

dans laquelle:

n et n' sont égaux à 2 ou 3,

R représente un méthoxy ou un hydroxy.

3. Sel d'ammonium quaternaire d'un dérivé de bis 7 H pyridocarbazole selon la revendication 1, caractérisé en ce que:

l'atome d'azote est en position 2,

R est en position 10 et représente l'un des groupes suivants: H, OH, Br, $OCH_3$, $OCOCH_3$, $OCOCH_2CH_3$, $O(CH_2)_2CH_3$, $NO_2$, $NH_2$;

R' représente H ou $CH_3$;

R" représente l'un des groupes suivants: H, CN, $CONH_2$, COOH, $CH_2NH_2$.

4. Sel d'ammonium quaternaire d'un dérivé de bis 7 H pyridocarbazole selon la revendication 1, caractérisé en ce que $R_1$ représente le groupe $CH_3$ ou $C_2H_5$.

5. Sel d'ammonium quaternaire d'un dérivé de bis 7 H pyridocarbazole selon la revendication 1, caractérisé en ce qu'il répond à la formule suivante:

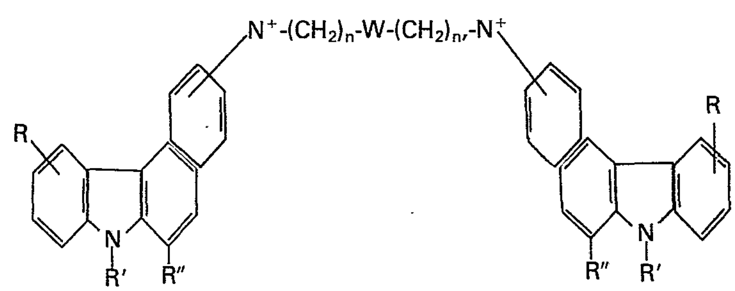

, $2A^-$

dans laquelle:

R, de préférence en position 10, représente le groupe méthoxy ou hydroxy;

n et n' prennent la valeur 2 ou 3;

W est représenté par la formule X-Y-X dans laquelle:

X représente la pipéridine et Y est le groupe $(CH_2)_3$ ou, X représente le groupe NH et Y est le groupe $(CH_2)_6$ ou le groupe $CH_2$-$(CHOH)_4$-$CH_2$.

6. Sel d'ammonium quaternaire d'un dérivé de bis 7 H pyridocarbazole selon la revendication 1, caractérisé en ce qu'il répond à la formule:

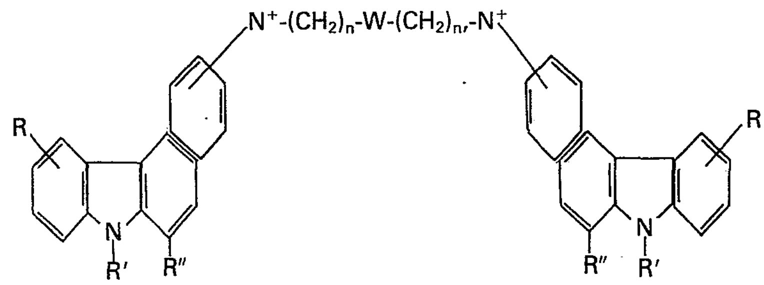

, $2A^-$

dans laquelle:

R, de préférence en position 10 représente un reste méthoxy ou hydroxy;

R′ représente un atome d'hydrogène ou un reste méthyle;

R″ représente un atome d'hydrogène;

W représente:

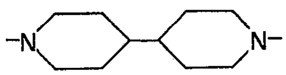

n et n′, de préférence égaux, prennent la valeur 2 ou 3.

7. Sel d'ammonium quaternaire d'un dérivé de bis 7 H pyridocarbazole selon l'une quelconque des revendications 1 à 6, caractérisé en ce que l'acide physiologiquement acceptable est choisi dans le groupe comprenant HCl, HBr, HI, l'acide acétique, l'acide lactique, gluconique et isothionique.

8. Sels d'ammonium quaternaire de dérivés de bis 7 H pyridocarbazoles selon la revendication 1, caractérisés en ce qu'ils répondent à l'une des formules suivantes:

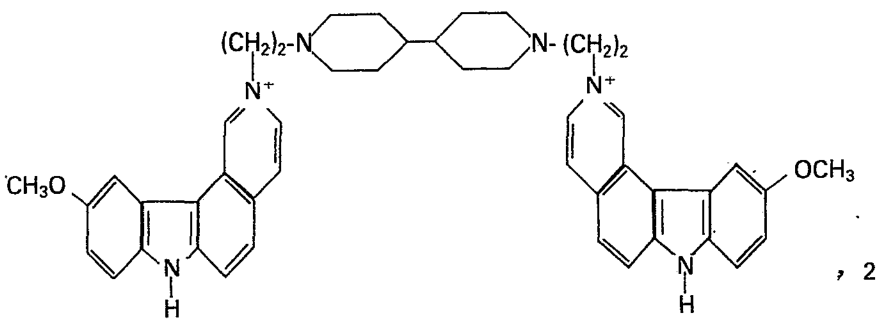

, 2 $Cl^-$

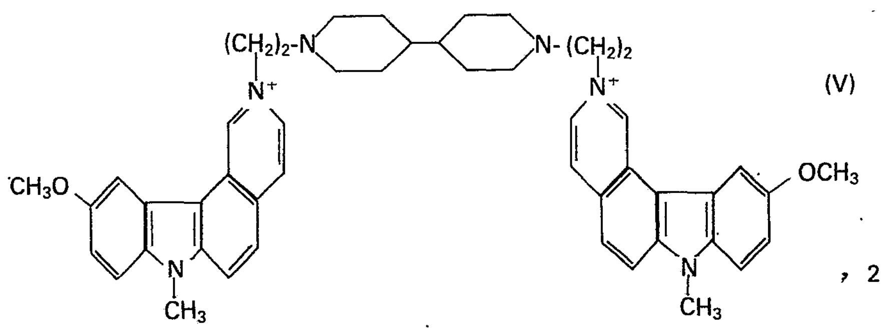

(V)

, 2 $Cl^-$

9. Sel d'ammonium quaternaire d'un dérivé de bis-7 H pyridocarbazole selon la revendication 1, caractérisé en ce qu'il répond à la formule:

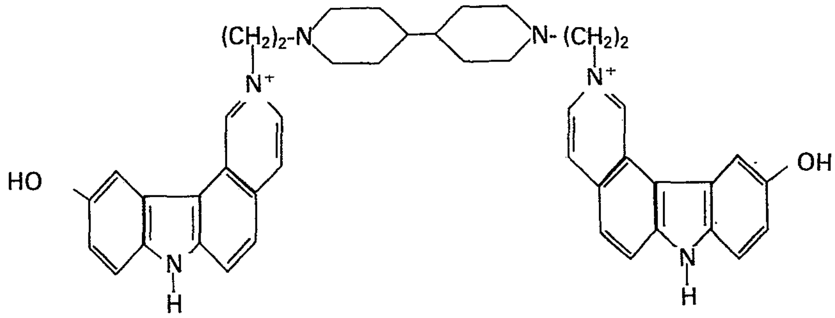

, 2 Cl

10. Sel d'ammonium quaternaire d'un dérivé de bis-7 H pyridocarbazole selon la revendication 1, caractérisé en ce qu'il répond à la formule:

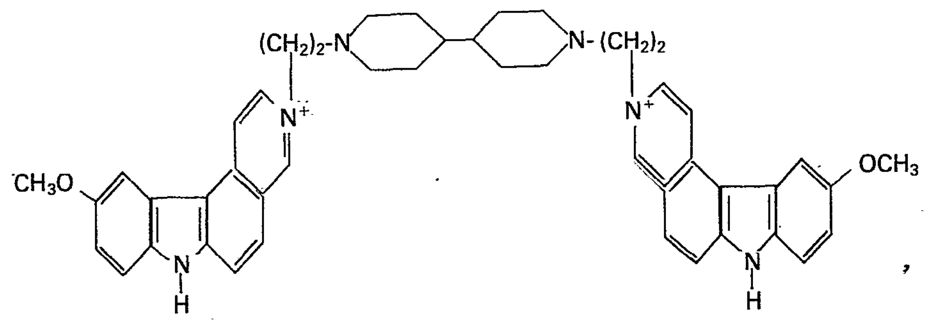

, 2 $Cl^-$

11. Composition pharmaceutique, à action antitumorale, caractérisée en ce qu'elle comprend en association avec un véhicule pharmaceutique un composé selon l'une quelconque des revendications 1 à 10.

**Claims**

1. Quaternary ammonium salt of a bis-7 H pyridocarbazole derivative, characterized by the following formula:

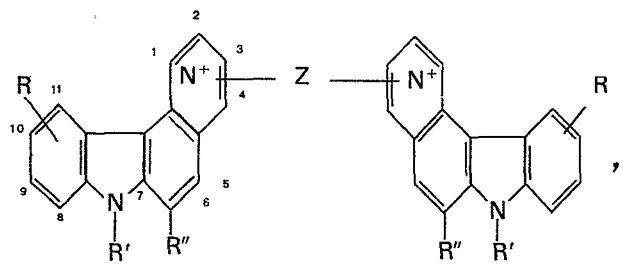

, $2A^-$

in which:

the nitrogen atom of the pyridil cycle occupies one of the 1 to 4 positions; and

Z is a molecular chain of formula:

$$-(CH_2)_n-W-(CH_2)_{n'}-$$

in which:

n and n' vary from 2 to 4:

W is selected from among the following groups:

$-(CH_2)_m-$

$NH-(CH_2)_p-(CHOH)_m-(CH_2)_{p'}$ -NH-

$NR_1-(CH_2)_p-(CHOH)_m-(CH_2)_{p'}$ $-NR_1-$

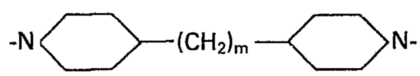

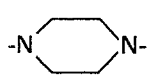

with m varying from 0 to 4, and p, p' having the values 1 or 2;

$NH-(CH_2)_{m'}$ -NH-

$NR_1-(CH_2)_{m'}$ $-NR_1-$

with m' varying from 2 to 8;

1,4-diamine benzene,

4,4'-diaminobiphenyl group,

4,4'-bipyridine,

decahydronaphtyridin group,

$R_1$ is an alkyl group comprising from 1 to 10 carbon atoms;

R which occupies any position on the corresponding ring is a hydrogen, a halogen, a nitro group, an amino group possibly substituted by alkyl rests comprising from 1 to 10 carbon atoms or acyl rests comprising from 2 to 10 carbon atoms or from 7 to 10 carbon atoms when these are aroyl rests, by aryl rests comprising from 6 to 10 carbon atoms, an OH group, possibly substituted by alkyl rests comprising from 1 to 12 carbon atoms, acyl comprising from 2 to 12 carbon atoms or from 7 to 12 carbon atoms, when these are aroyl rests, by aryl rests comprising from 6 to 12 carbon atoms;

R' is a hydrogen, an alkyl rest comprising from 1 to 10 carbon atoms or an aryl rest comprising from 6 to 12 carbon atoms, or acyl comprising from 2 to 10 carbon atoms or from 7 to 12 carbon atoms when it is an aroyl group;

R'' is a hydrogen atom,

a cyano, formyl, hydroxymethyl, radical possibly esterified by an aliphatic carboxylic acid from 1 to 10 carbon atoms,

an aminomethyl radical possibly substituted on

the amino by an alkyl or acyl group from 1 to 10 carbon atoms,

a carboxyl group possibly esterified by an aliphatic alcohol from 1 to 10 carbon atoms,

or an amido radical;

$A^-$ being the anion of a physiologically acceptable acid.

2. Quaternary ammonium salt of a bis-7 H pyridocarbazole derivative according to claim 1, characterized in that the nitrogen atoms of the pyridil cycles are in the 2 position and by the fact the Z is a chain of formula:

$$-(CH_2)_n-W-(CH_2)_{n'}-$$

in which:

n and n′ are equal to 2 or 3,

R represents a methoxy or a hydroxy.

3. Quaternary ammonium salt of a bis-7 H pyridocarbazole derivative according to claim 1, characterized in that:

the nitrogen atom is in the 2 position;

R is in the 10 position and represents one of the following groups: H, OH, Br, $OCH_3$, $OCOCH_3$, $OCOCH_2CH_3$, $O(CH_2)_2CH_3$, $NO_2$, $NH_2$;

R′ represents H or $CH_3$;

R″ represents one of the following groups: H, CN, $CONH_2$, COOH, $CH_2NH_2$.

4. Quaternary ammonium salt of a bis-7 H pyridocarbazole derivative according to claim 1, characterized in that $R_1$ represents $CH_3$ or $C_2H_5$.

4. Quaternary ammonium salt of a bis-7 H pyridocarbazole derivative according to claim 1, characterized in that $R_1$ represents $CH_3$ or $C_2H_5$.

5. Quaternary ammonium salt of a bis-7 H pyridocarbazole derivative according to claim 1, characterized in that it can be represented by the following formula:

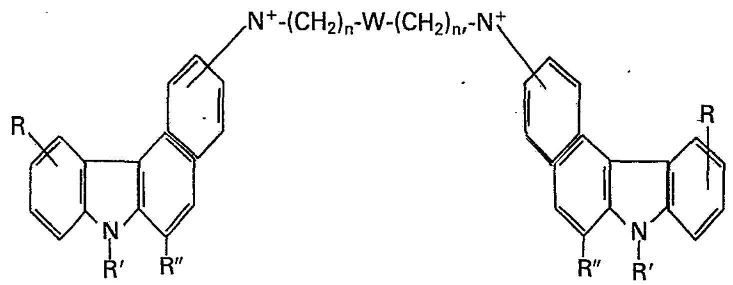

, $2A^-$

in which:

R, preferably in the 10 position, represents the methoxy or hydroxy group;

n and n′ have the value 2 or 3;

W is represented by the formula X-Y-X in which:

X represents piperidine and Y is the $(CH_2)_3$ group or X represents the NH group and Y is the $(CH_2)_6$ group or the $CH_2$-$(CHOH)_4$-$CH_2$ group.

6. Quaternary ammonium salt of a bis-7 H pyridocarbazole derivative according to claim 1, characterized in that it can be represented by the following formula:

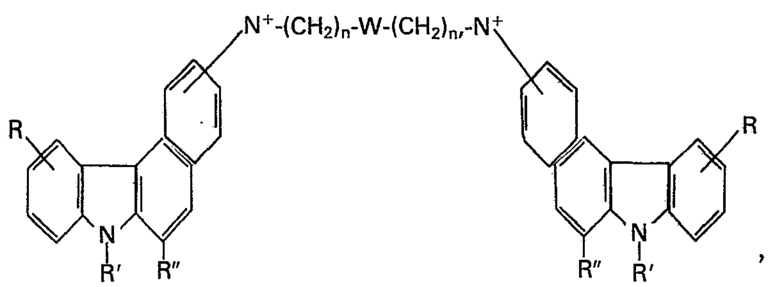

, $2A^-$

in which:

R, preferably in the 10 position, represents a methoxy or hydroxy group;

R′ represents a hydrogen atom or a methyl group;

R″ represents a hydrogen atom;

W represents:

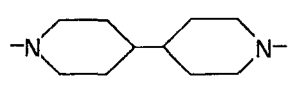

n and n′, preferably equal, have the value 2 or 3.

7. Quaternary ammonium salt of a bis-7 H pyridocarbazole derivative according to anyone of claims 1 to 6, characterized in that the physiologically acceptable acid is selected from among the group comprising HCl, HBr, HI, acetic acid, lactic acid, gluconic and isothionic acids.

8. Quaternary ammonium salts of bis-7 H pyridocarbazole derivatives according to claim 1, characterized in that they can be represented one of the following formula:

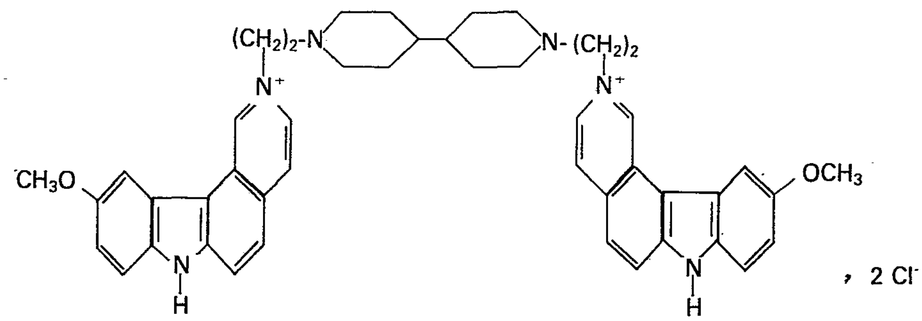

, 2 $Cl^-$

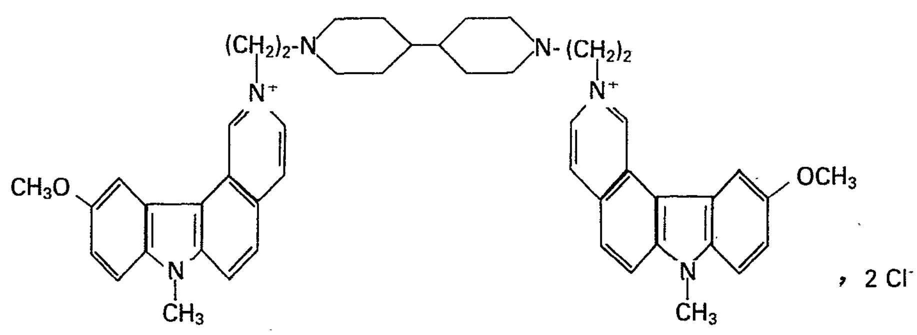

, 2 $Cl^-$

9. Quaternary ammonium salt of a bis-7 H pyridocarbazole derivative according to claim 1, characterized in that it can be represented by the formula:

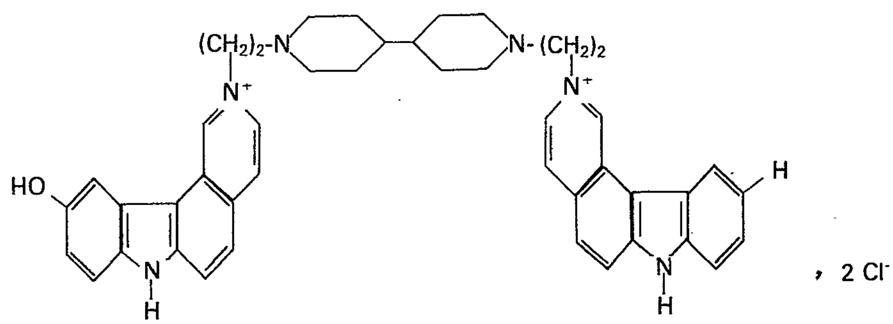

, 2 $Cl^-$

10. Quaternary ammonium salt of a bis-7 H pyridocarbazole derivative according to claim 1, characterized in that it can be represented by the formula:

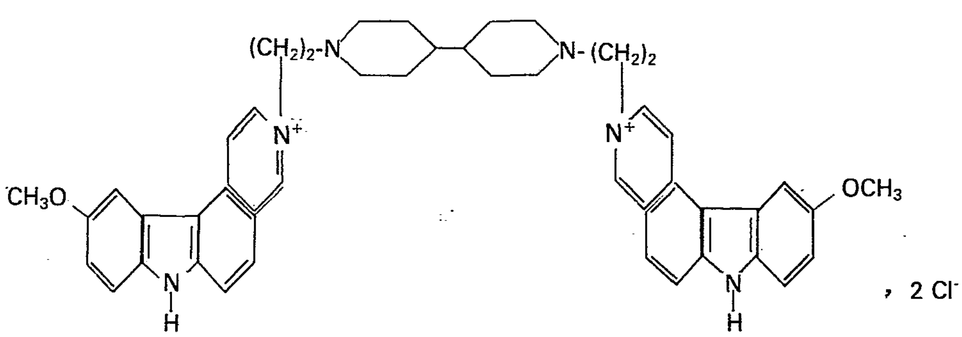

, 2 $Cl^-$

11. Pharmaceutical composition with antitumoral action, characterized in that it comprises in association with a pharmaceutical vehicle a compound according to anyone of claims 1 to 10.

**Patentansprüche**

1. Quartäres Ammoniumsalz einer bis-7 H Pyridocarbazolverbindung, gekennzeichnet durch folgende Formel:

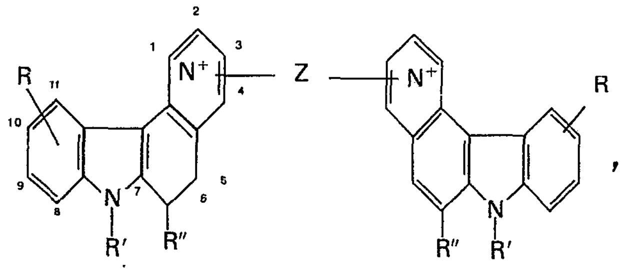

, $2A^-$

in der:

das Stickstoffatom des Pyridilringes eine der Stellungen 1 bis 4 einnimmt, und

Z eine Molekularkette folgender Formel ist:

$$-(CH_2)_n-W-(CH_2)_{n'}-$$

in der:

n und n' bedeuten von 2 bis 4:

W unter den folgenden Gruppen ausgewählt ist:

$-(CH_2)_m-$

$-NH-(CH_2)_p-(CHOH)_m-(CH_2)_{p'}$ -NH-

$-NR_1-(CH_2)_p-(CHOH)_m-(CH_2)_{p'}$ $-NR_1-$

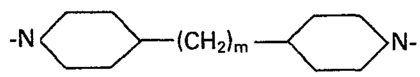

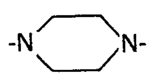

mit m bedeutend von 0 bis 4, und wobei p, p' 1 oder 2 bedeuten;

$-NH-(CH_2)_{m'}$ -NH-

$-NR_1-(CH_2)_{m'}$ $-NR_1-$

mit m' bedeutend von 2 bis 8;

das 1,4-Diamine-benzol,

die Gruppe 4,4'-Diaminobiphenyl,

das 4,4'-Bipyridin,

die Gruppe Decahydronaphthyridin,

$R_1$ eine Alkylgruppe ist mit 1 bis 10 Kohlenstoffatomen;

R das eine beliebige Stellung in dem entsprechenden Ring einnehmen kann, ein Wasserstoffatom, ein Halogenatom, eine Nitrogruppe, eine Aminogruppe ist, gegebenenfalls substituiert durch Alkylreste mit 1 bis 10 Kohlenstoffatomen, oder Acylreste mit 2 bis 10 Kohlenstoffatomen oder mit 7 bis 10 Kohlenstoffatomen, wenn es sich um Aroylreste handelt, durch Arylreste mit 6 bis 10 Kohlenstoffatomen, eine OH-Gruppe, gegebenenfalls substituiert durch Alkylreste mit 1 bis 12 Kohlenstoffatomen, Acyl mit 2 bis 12 Kohlenstoffatomen oder 7 bis 12 Kohlenstoffatomen, wenn es sich um Aroylreste handelt, durch Arylreste mit 6 bis 12 Kohlenstoffatomen

R' ist ein Wasserstoffatom, ein Alkylrest mit 1 bis 10 Kohlenstoffatomen oder ein Arylrest mit 6 bis 12 Kohlenstoffatomen, oder Acyl mit 2 bis 10 Kohlenstoffatomen oder 7 bis 12 Kohlenstoffatomen, wenn es sich um einen Aroylrest handelt;

R" ist ein Wasserstoffatom,

ein Cyanoradikal, ein Formylradikal, ein Hydroxymethylradikal gegebenenfalls verestert durch eine aliphatische Carbonsäure mit 1 bis 10 Kohlenstoffatomen,

ein Aminomethylradikal gegebenenfalls substituiert im Amino durch eine Alkylgruppe oder Acylgruppe mit 1 bis 10 Kohlenstoffatomen

ein Carboxylradikal gegebenenfalls verestert durch einen aliphatischen Alkohol mit 1 bis 10 Kohlenstoffatomen,

oder ein Radikal Amido

wobei $A^-$ das Anion einer physiologisch verträglichen Säure darstellt.

2. Quartäres Ammoniumsalz einer bis-7 H Pyridocarbazolverbindung nach Anspruch 1, dadurch gekennzeichnet, dass die Stickstoffatome der Pyridylringe in Stellung 2 sind und dass Z eine Kette folgender Formel ist:

$$-(CH_2)_n-W-(CH_2)_{n'}-$$

in der:

n und n' gleich 2 oder 3 sind,

R eine Methoxy oder ein Hydroxy darstellt.

3. Quartäres Ammoniumsalz einer bis-7 H Pyridocarbazolverbindung nach Anspruch 1, dadurch gekennzeichnet, dass:

das Stickstoffatom sich in Stellung 2 befindet,

R Stellung 10 einnimmt und eine der folgenden Gruppen darstellt: H, OH, Br, $OCH_3$, $OCOCH_3$, $OCOCH_2CH_3$,$O(CH_2)_2CH_3$, $NO_2$, $NH_2$;

R' H oder $CH_3$ darstellt;

R" eine der folgenden Gruppen darstellt: H, CN, $CONH_2$, COOH, $CH_2NH_2$.

4. Quartäres Ammoniumsalz einer bis-7 H Pyridocarbazolverbindung nach Anspruch 1, dadurch gekennzeichnet, dass $R_1$ die Gruppe $CH_3$ oder $C_2H_5$ darstellt.

5. Quartäres Ammoniumsalz einer bis-7 H Pyridocarbazolverbindung nach Anspruch 1, dadurch gekennzeichnet, dass es folgender Formel entspricht:

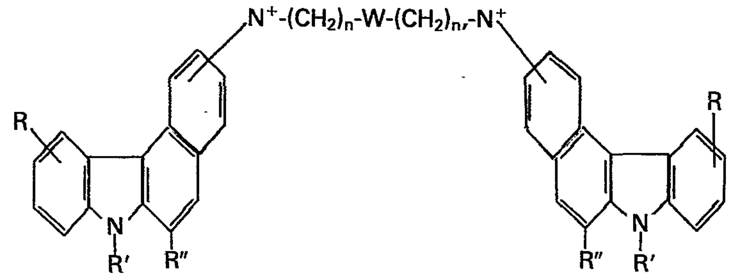

, $2A^-$

in der:

R, vorzugsweise in Stellung 10, die Gruppe Methoxy oder Hydroxy darstellt;

n und n' für 2 oder 3 stehen;

W der Formel X-Y-X entspricht, in der:

X Piperidin und Y die Gruppe $(CH_2)_3$ darstellt, oder X stellt die Gruppe NH und Y die Gruppe $(CH_2)_6$ oder die Gruppe $CH_2$-$(CHOH)_4$-$CH_2$ dar.

6. Quartäres Ammoniumsalz einer bis-7 H Pyridocarbazolverbindung nach Anspruch 1, dadurch gekennzeichnet, dass es folgender Formel entspricht:

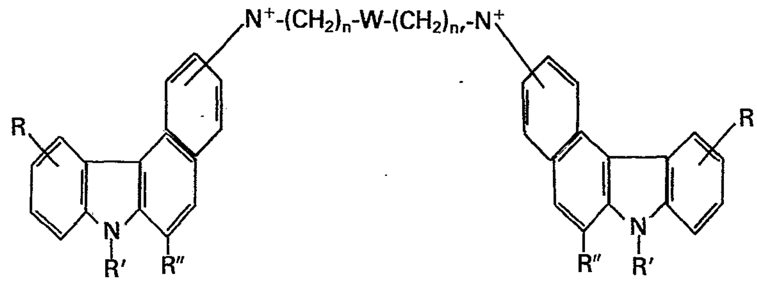

, $2A^-$

in der:

R, vorzugsweise in Stellung 10, einen Rest Methoxy oder Hydroxy darstellt;

R' ein Wasserstoffatom oder ein Methylrest darstellt;

R'' ein Wasserstoffatom darstellt;

W:

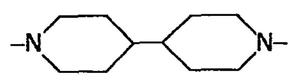

darstellt.

n und n', vorzugsweise gleich, für 2 oder 3 stehen.

7. Quartäres Ammoniumsalz einer bis-7 H Pyridocarbazolverbindung nach einem beliebigen der Ansprüche 1 bis 6, dadurch gekennzeichnet, dass die physiologisch verträgliche Säure aus der Gruppe umfassend HCl, HBr, HI, Essigsäure, Milchsäure, Glukonsäure und Isothionsäure gewählt wird.

8. Quartäre Ammoniumsalze von bis-7 H Pyridocarbazolverbindungen nach Anspruch 1, dadurch gekennzeichnet, dass sie einer der folgenden Formeln entsprechen:

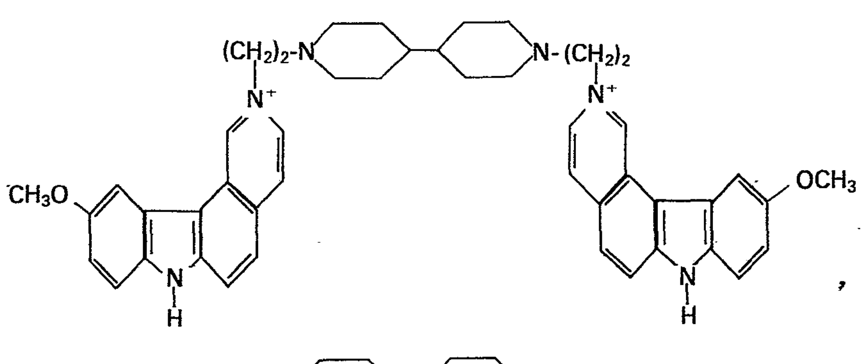

, 2 $Cl^-$

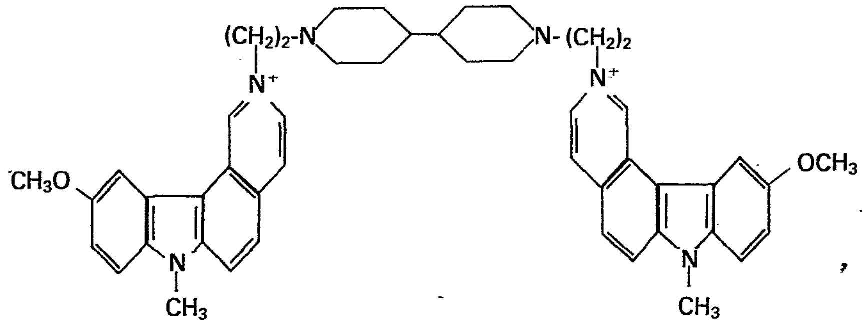

, 2 $Cl^-$

9. Quartäres Ammoniumsalz einer bis-7 H Pyridocarbazolverbindung nach Anspruch 1, dadurch gekennzeichnet, dass es der Formel

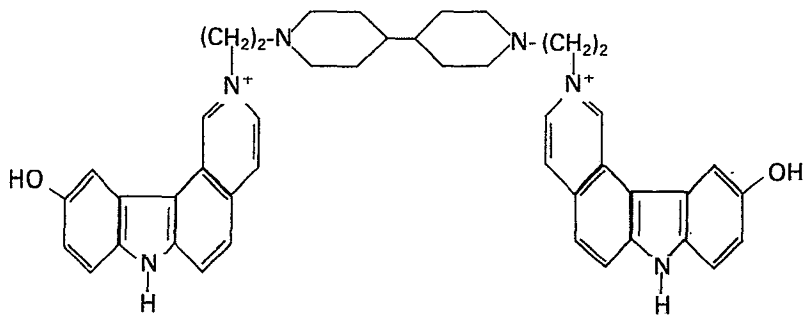

, 2 $Cl^-$

entspricht.

10. Quartäres Ammoniumsalz einer bis-7 H Pyridocarbazolverbindung nach Anspruch 1, dadurch gekennzeichnet, dass es der Formel:

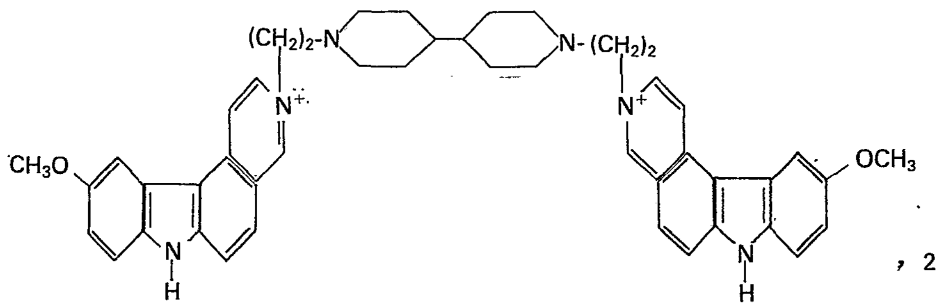

, 2 $Cl^-$

entspricht.

11. Pharmazeutische Zusammensetzung mit Antitumorwirkung, dadurch gekennzeichnet, dass sie in Verbindung mit einem pharmazeutischen Träger eine Verbindung entsprechend einem beliebigen der Ansprüche 1 bis 10 enthält.

Fig.1a.

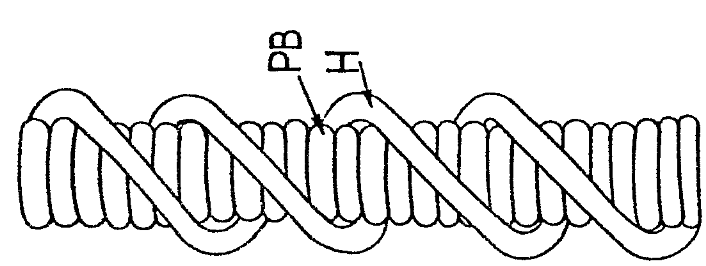


Fig.1b.

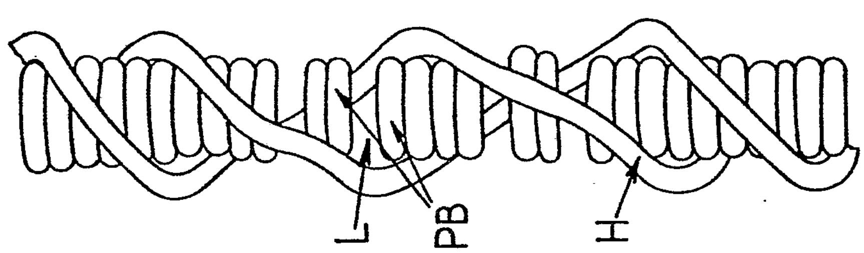


Fig.1c.

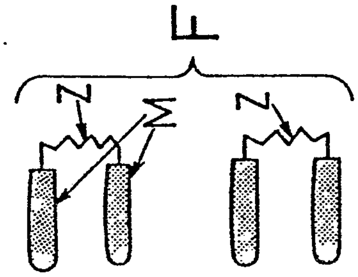


Fig.1d.

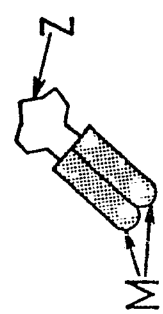


Fig.1e.

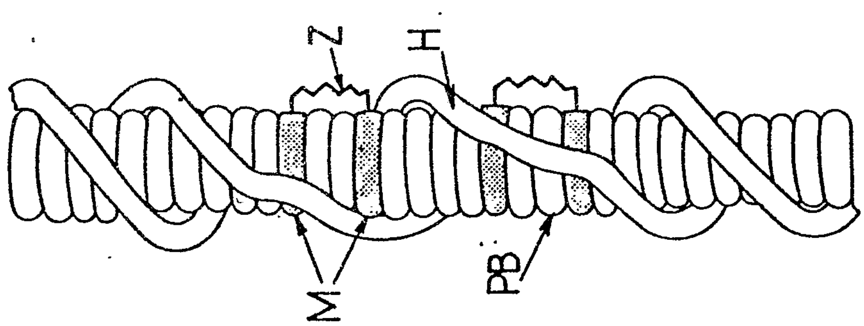

Fig . 2.

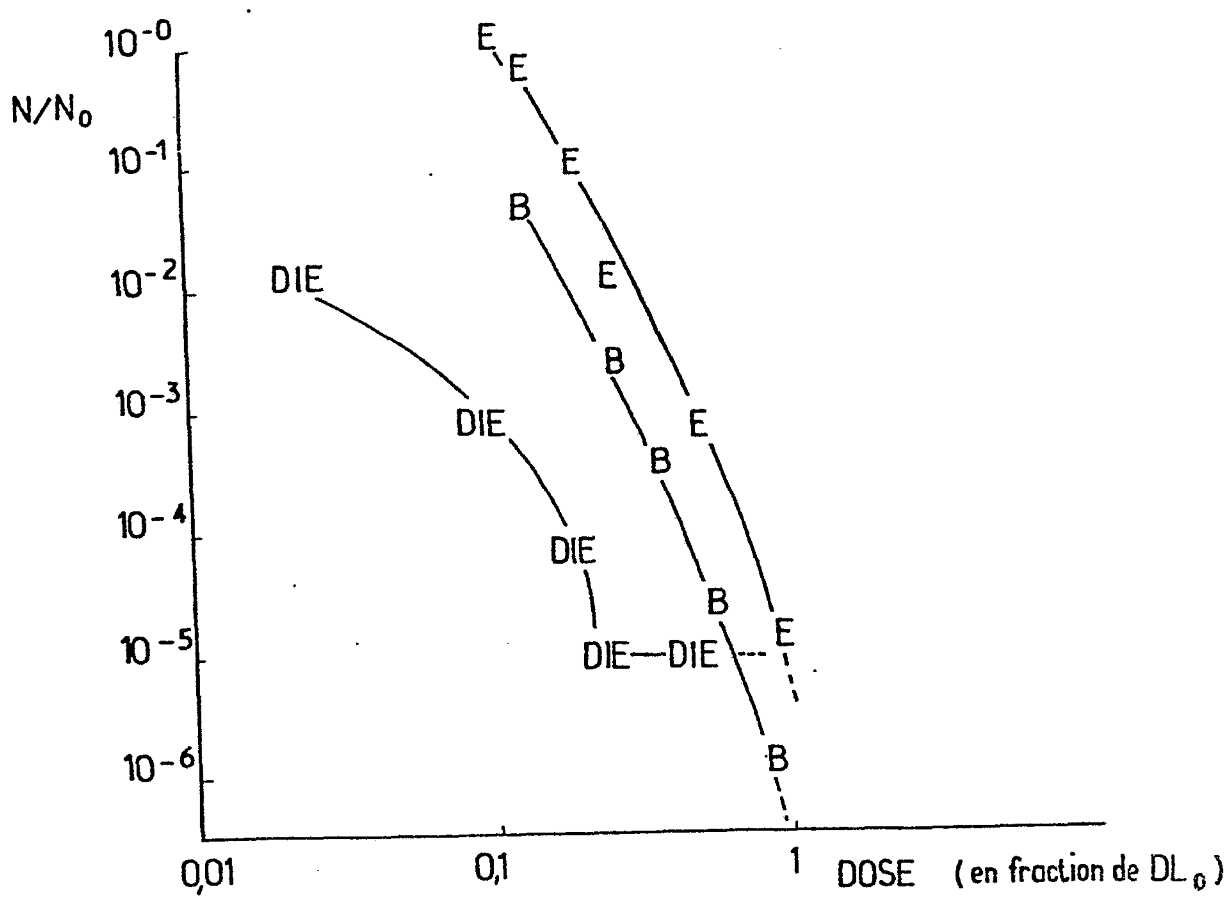

$10^{-0}$
N/No
$10^{-1}$
$10^{-2}$
$10^{-3}$
$10^{-4}$
$10^{-5}$
$10^{-6}$
E
E
E
E
E
E
B
B
B
B
B
DIE
DIE
DIE
DIE—DIE
0,01
0,1
1
DOSE (en fraction de $DL_0$)